(19) Europäisches Patentamt / European Patent Office / Office européen des brevets

(11) **EP 4 578 386 B1**

(12) # EUROPEAN PATENT SPECIFICATION

(45) Date of publication and mention
of the grant of the patent:
**22.04.2026   Bulletin 2026/17**

(21) Application number: **24221319.7**

(22) Date of filing: **18.12.2024**

(51) International Patent Classification (IPC):
**A61B 5/271** (2021.01)          **A61B 5/291** (2021.01)
**A61B 5/374** (2021.01)          **A61B 5/00** (2006.01)

(52) Cooperative Patent Classification (CPC):
**A61B 5/291; A61B 5/0531; A61B 5/271;
A61B 5/374; A61B 5/6803; A61B 5/6843;
A61B 5/7203; A61B 5/7221**

(54) **METHOD FOR CONTROLLING SIGNAL ACQUISITION ELECTRODE AND EEG SIGNAL ACQUISITION DEVICE**

VERFAHREN ZUR STEUERUNG EINER SIGNALERFASSUNGSELEKTRODE UND EEG-SIGNALERFASSUNGSVORRICHTUNG

PROCÉDÉ DE COMMANDE D'ÉLECTRODE D'ACQUISITION DE SIGNAL ET DISPOSITIF D'ACQUISITION DE SIGNAL D'ÉLECTROENCÉPHALOGRAMME (EEG)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **26.12.2023   CN 202311810635
26.12.2023   CN 202311808091
26.12.2023   CN 202311814246**

(43) Date of publication of application:
**02.07.2025   Bulletin 2025/27**

(73) Proprietor: **Kingfar International Inc.
Haidian District
Beijing 100085 (CN)**

(72) Inventors:
• **ZHAO, Qichao
Beijing, 100085 (CN)**
• **YANG, Ran
Beijing, 100085 (CN)**

(74) Representative: **Range, Christopher William
Withers & Rogers LLP
2 London Bridge
London SE1 9RA (GB)**

(56) References cited:
WO-A1-2013/038285          WO-A1-2021/181395
CN-A- 112 315 483          US-A1- 2009 259 137
US-A1- 2011 015 503          US-A1- 2014 051 044
US-A1- 2019 000 338

## Description

### CROSS-REFERENCE TO RELATED APPLICATION

The present application claims priority to Chinese Patent Application No. 202311814246.8, entitled "ELECTROENCEPHALOGRAM SIGNAL ACQUISITION APPARATUS AND THE METHOD FOR CONTROLLING SIGNAL ACQUISITION ELECTRODE THEREOF", and filed on December 26, 2023; Chinese Patent Application No. 202311810635.3, entitled "HUMAN INTELLIGENCE-BASED IMPEDANCE DETECTION METHODAND APPARATUS, ELECTRONIC DEVICE, AND MEDIUM", and filed on December 26, 2023; and Chinese Patent Application No. 202311808091.7, entitled "HUMAN INTELLIGENCE-BASED ELECTROENCEPHALOGRAM DATA VERIFICATION METHOD AND APPARATUS, AND DEVICE AND MEDIUM", and filed on December 26, 2023.

### FIELD

The present disclosure relates to the field of electroencephalogram (EEG) signal acquisition technologies, and more particularly, to a method for controlling a signal acquisition electrode, an EEG signal acquisition apparatus, and a medium.

### BACKGROUND

EEG is a bioelectric signal generated when the brain is active and information is transmitted between a large number of neurons. EEG can be used for disease diagnosis and treatment or scientific research. Before using EEG for the disease diagnosis and treatment (or the scientific research), it is necessary to collect the EEG through an EEG signal acquisition apparatus.

An EEG signal acquisition apparatus in the related art includes a cap and a signal acquisition electrode disposed at the cap. Before collecting an EEG signal, a user can wear the cap. After the user wears the cap, the staff can manually adjust the signal acquisition electrode to allow the signal acquisition electrode to be in full contact with a skin of the user's head to avoid poor quality of acquired EEG due to insufficient contact. After the adjustment is completed, the EEG signal acquisition apparatus can be started to acquire the user's EEG signal.

Thus, the EEG signal acquisition apparatus in the related art has a low intelligence degree. WO2013038285A1 discloses an EEG system including a first EEG electrode, providing a first EEG signal and a first impedance signal, and a second EEG electrode, providing a second EEG signal and a second impedance signal, a determination unit for determining an impedance difference value indicating the impedance difference between the electrodes, and a control unit for controlling the first and/or the second electrode depending on the impedance difference value. The control unit fuses the information received from different electrodes and uses it for automatically adjusting the pressure in each electrode.

US2019000338A1 discloses an EEG system including an EEG electrode configured to be positioned for contacting skin of the user, an actuator operatively coupled to the EEG electrode and configured to move the electrode in at least two dimensions, including an axial dimension and a lateral dimension to enable the EEG electrode to contact the skin at different locations, and one or more physical processors operatively connected with the EEG electrode and the actuator. The one or more physical processors are configured to: obtain an impedance signal from the EEG electrode; and actuate the actuator to move the EEG electrode based on a comparison of the obtained impedance signal with an impedance threshold. US2011015503A1 discloses an EEG processing unit including an automated connectivity determination apparatus which can use pressure-sensitive electrode placement ensuring proper contact with Patient's scalp and also automatically verifies electrode placement via measurements of electrode impedance through automated impedance checking.

CN112315483A discloses an electrode self-adjusting method and device based on electroencephalogram signal collection. The electrode self-adjusting method includes controlling a target electrode at a specified position to move in a direction close to the skin of a user at a first preset speed, and obtaining the detection pressure borne by the target electrode; comparing the detection pressure with a first pressure threshold value, and if the detection pressure is smaller than the first pressure threshold value, obtaining real-time impedance of the target electrode; and comparing the real-time impedance with a preset impedance threshold value, and if the real-time impedance is smaller than or equal to the preset impedance threshold value, stopping the target electrode from moving to complete the adjustment of the target electrode.

### SUMMARY

The present disclosure provides a method for controlling a signal acquisition electrode, an EEG signal acquisition apparatus, a medium, and an electronic device, capable of solving a problem of the low intelligence degree of an EEG signal acquisition apparatus in the related art. The technical solutions according to embodiments of the present disclosure are as follows.

The invention is set out in the appended set of claims.

In a first aspect of the disclosure, provided is a method for controlling a signal acquisition electrode. The signal acquisition electrode is movably connected to a wearable component of an EEG signal acquisition apparatus. The wearable component is adapted to be worn on a head of a user, and the EEG signal acquisition apparatus further includes a pushing component. The method includes: controlling, when a contact impedance between the signal acquisition electrode and the head is greater than or equal to a first impedance threshold, the pushing component to push the signal acquisition electrode to move towards the head to acquire an EEG signal by using the signal acquisition electrode.

In some embodiments, the controlling the pushing component to push the signal acquisition electrode to move towards the head includes: determining, from a correspondence between impedances and intensities, a reference pushing intensity corresponding to the contact impedance; and controlling the pushing component to push, in accordance with the reference pushing intensity, the signal acquisition electrode to move towards the head. A plurality of impedances are recorded in the correspondence, and any two of the plurality of impedances differ from each other.

In some embodiments, the controlling the pushing component to push the signal acquisition electrode to move towards the head further includes: determining a target pushing intensity of the pushing component based on a difference between the first impedance threshold and the contact impedance; and controlling the pushing component to push, in accordance with the target pushing intensity, the signal acquisition electrode to move towards the head.

In some embodiments, said determining the target pushing intensity of the pushing component based on the difference between the first impedance threshold and the contact impedance includes: processing the difference between the first impedance threshold and the contact impedance with a proportional-integral-derivative algorithm, to obtain the target pushing intensity of the pushing component.

In some embodiments, the EEG signal acquisition apparatus includes a plurality of signal acquisition electrodes; and the method further includes: acquiring, by using the plurality of signal acquisition electrodes, the EEG signal in response to a contact impedance of each of a target number of signal acquisition electrodes among the plurality of signal acquisition electrodes satisfying a target condition. The target number is smaller than or equal to a total number of the plurality of signal acquisition electrodes and is greater than a number threshold. The target condition includes a duration during which the contact impedance is smaller than the first impedance threshold reaching a target duration.

In some embodiments, the method further includes: detecting the contact impedance. The detecting the contact impedance includes: applying, by using a first alternating-current constant current source, an electrical signal to the signal acquisition electrode, and detecting first impedance data between the signal acquisition electrode and the head; applying, by using a second alternating-current constant current source, an electrical signal to the signal acquisition electrode, and detecting second impedance data between the signal acquisition electrode and the head; determining, based on a data distribution feature of the first impedance data and a data distribution feature of the second impedance data, a first confidence level for the first impedance data and a second confidence level for the second impedance data; and performing data fusion on the first impedance data and the second impedance data based on the first confidence level and the second confidence level, to obtain the contact impedance.

In some embodiments, a frequency of the electrical signal applied by the first alternating-current constant current source differs from a frequency of the electrical signal applied by the second alternating-current constant current source, the first alternating-current constant current source is provided by an integrated chip configured to measure the EEG signal, and the second alternating-current constant current source is provided by a Wien bridge self-excited oscillation circuit configured to generate a sinusoidal wave; and/or the first alternating-current constant current source applies the electrical signal to the signal acquisition electrode and a reference electrode, and the second alternating-current constant current source applies the electrical signal to the signal acquisition electrode and the reference electrode; and/or impedance data is detected at a plurality of first time points, and impedance data detected at adjacent time points are selected from the impedance data detected at the plurality of first time points as the first impedance data; and impedance data is detected at a plurality of second time points, and impedance data detected at adjacent time points are selected from the impedance data detected at the plurality of second time point as the second impedance data, the first impedance data being normally distributed, and the second impedance data being normally distributed.

In some embodiments, the data distribution feature includes data discretization feature; and the determining, based on the data distribution feature of the first impedance data and the data distribution feature of the second impedance data, the first confidence level of the first impedance data and the second confidence level of the second impedance data includes: determining a first deviation value of the first impedance data based on a data discretization feature of the first impedance data; determining a second deviation value of the second impedance data based on a data discretization feature of the second impedance data; and determining the first confidence level and the second confidence level based on the first deviation value and the second deviation value.

In some embodiments, the determining the first confidence level and the second confidence level based on the first deviation value and the second deviation value includes: determining the first confidence level based on the first deviation value and the second deviation value; and determining the second confidence level based on the first confidence level and a predetermined constraint condition, the predetermined constraint condition including a sum of the first confidence level and the second confidence level being 1.

In some embodiments, the first deviation value includes a first variance of first impedance data conforming to a normal distribution, and the second deviation value includes a second variance of second impedance data conforming to a normal distribution; and the determining the first confidence level based on the first deviation value and the second deviation value includes: determining a functional relationship between a fused variance and the first confidence level based on the first variance and the second variance, where the fusion variance is a variance of fused impedance data, the fused impedance data representing a fusion relationship between the first impedance data and the second impedance data; and taking a derivative of the first confidence level based on the functional relationship between the fused variance and the first confidence level, and obtaining the first confidence level when the fusion variance is minimized.

In some embodiments, said performing the data fusion on the first impedance data and the second impedance data based on the first confidence level and the second confidence level, to obtain the contact impedance includes: obtaining first data by multiplying the first confidence level by the first impedance data, obtaining second data by multiplying the second confidence level by the second impedance data, and adding the first data and the second data to obtain fused impedance data; determining, when the fused impedance data conforms to a normal distribution, a mean of the fused impedance data as the contact impedance.

In some embodiments, the method further includes: verifying the EEG signal subsequent to the EEG signal having been acquired by the signal acquisition electrode, where said verifying the EEG signal includes: determining, based on a power frequency interference signal that interferes with the EEG signal, first invalidity data for characterizing validity of the EEG signal; determining, based on the contact impedance, second invalidity data for characterizing validity of the EEG signal; and performing data fusion on the first invalidity data and the second invalidity data, to obtain comprehensive invalidity data.

In some embodiments, the determining, based on the power frequency interference signal that interferes with the EEG signal, the first invalidity data for characterizing the validity of the EEG signal includes: determining the first invalidity data based on a signal intensity of the power frequency interference signal and a first predetermined relationship, the first predetermined relationship characterizing a relationship between the signal intensity of the power frequency interference signal and the first invalidity data.

In some embodiments, the signal intensity of the power frequency interference signal is obtained by: filtering the EEG signal based on a predetermined frequency to obtain a filtered EEG signal, the predetermined frequency including 50 Hz; obtaining the power frequency interference signal based on a difference between the EEG signal before the filtering and the filtered EEG signal; and calculating a root mean square of the power frequency interference signal as the signal intensity of the power frequency interference signal; and/or the first predetermined relationship includes: when the signal intensity of the power frequency interference signal is smaller than a first signal intensity threshold, a trend in which the first invalidity data increases as the signal intensity of the power frequency interference signal increases being a first trend; when the signal intensity of the power frequency interference signal is greater than or equal to the first signal intensity threshold and smaller than a second signal intensity threshold, a trend in which the first invalidity data increases as the signal intensity of the power frequency interference signal increases being a second trend; and when the signal intensity of the power frequency interference signal is greater than or equal to the second signal intensity threshold and smaller than a third signal intensity threshold, a trend in which the first invalidity data increases as the signal intensity of the power frequency interference signal increases being a third trend. The first signal intensity threshold is smaller than the second signal intensity threshold, and the second signal intensity threshold is smaller than the third signal intensity threshold; and the first trend is smaller than the second trend, and the second trend is smaller than the third trend.

In some embodiments, the determining, based on the contact impedance, the second invalidity data for characterizing the validity of the EEG signal includes: determining the second invalidity data based on the contact impedance and a second predetermined relationship. The second predetermined relationship characterizes a relationship between the contact impedance and the second invalidity data; and the second predetermined relationship includes: when the contact impedance is smaller than a first impedance threshold, a trend in which the second invalidity data increases as the contact impedance increases being a fourth trend; when the contact impedance is greater than or equal to the first impedance threshold and smaller than a second impedance threshold, a trend in which the second invalidity data increases as the contact impedance increases being a fifth trend; when the contact impedance is greater than or equal to the second impedance threshold and smaller than a third impedance threshold, a trend in which the second invalidity data increases as the contact impedance increases being a sixth trend. The first impedance threshold is smaller than the second impedance threshold, and the second impedance threshold is smaller than the third impedance threshold; and the fourth trend is smaller than the fifth trend, and the fifth trend is smaller than the sixth trend.

In some embodiments, said performing the data fusion on the first invalidity data and the second invalidity data to obtain the comprehensive invalidity data includes:

determining a first weight of the first invalidity data; determining a second weight of the second invalidity data; obtaining first data by multiplying the first invalidity data by the first weight, obtaining second data by multiplying the second invalidity data by the second weight, and adding the first data and the second data to obtain the comprehensive invalidity data. When the signal intensity of the power frequency interference signal is greater than a predetermined threshold, the first weight is greater than the second weight; when the signal intensity of the power frequency interference signal is smaller than or equal to the predetermined threshold, the first weight is smaller than or equal to the second weight; and a sum of the first weight and the second weight is 1.

In a second aspect, provided is an EEG signal acquisition apparatus. The apparatus includes a wearable component, a pushing component, and a signal acquisition electrode. The wearable component is adapted to be worn on a head of a user and has a mounting cavity. The pushing component is disposed in the mounting cavity. The signal acquisition electrode is movably connected to the wearable component. The pushing component is configured to push the signal acquisition electrode to move towards the head when contact impedance between the signal acquisition electrode and the head is greater than or equal to a first impedance threshold.

In some embodiments, the pushing component is further configured to push, when the contact impedance between the signal acquisition electrode and the head is greater than or equal to the first impedance threshold, the signal acquisition electrode to move towards the head in accordance with a reference pushing intensity. The reference pushing intensity is determined based on the contact impedance and a correspondence between impedances and intensities, a plurality of impedances are recorded in the correspondence, and any two of the plurality of impedances differ from each other.

In some embodiments, the pushing component is an airbag configured to be inflated when the contact impedance between the signal acquisition electrode and the head is greater than or equal to the first impedance threshold, to push the signal acquisition electrode to move towards the head; or the pushing component is a retractable assembly configured to be in an extended state when the contact impedance between the signal acquisition electrode and the head is greater than or equal to the first impedance threshold, to push the signal acquisition electrode to move towards the head.

In some embodiments, the signal acquisition electrode is a needle-shaped electrode; and the apparatus further includes: an inflation component connected to the airbag, the inflation component being configured to inflate the airbag when the contact impedance between the signal acquisition electrode and the head is greater than or equal to the first impedance threshold; and/or a controller configured to control the pushing component to push the signal acquisition electrode to move towards the head when the contact impedance between the signal acquisition electrode and the head is greater than or equal to the first impedance threshold.

In a third aspect, provided is a human intelligence-based impedance detection method. The method includes: applying, by using a first alternating-current constant current source, an electrical signal to a signal acquisition electrode, and detecting first impedance data between the signal acquisition electrode and a head; applying, by using a second alternating-current constant current source, an electrical signal to the signal acquisition electrode, and detecting second impedance data between the signal acquisition electrode and the head; determining, based on a data distribution feature of the first impedance data and a data distribution feature of the second impedance data, a first confidence level of the first impedance data and a second confidence level of the second impedance data; and performing data fusion on the first impedance data and the second impedance data based on the first confidence level and the second confidence level, to obtain a contact impedance.

In some embodiments, a frequency of the electrical signal applied by the first alternating-current constant current source differs from a frequency of the electrical signal applied by the second alternating-current constant current source. The first alternating-current constant current source is provided by an integrated chip configured to measure the EEG signal, and the second alternating-current constant current source is provided by a Wien bridge self-excited oscillation circuit configured to generate a sinusoidal wave.

In some embodiments, impedance data is detected at a plurality of first time points, and impedance data detected at adjacent time points are selected from the impedance data detected at the plurality of first time points as the first impedance data; and impedance data is detected at a plurality of second time points, and impedance data detected at adjacent time points are selected from the impedance data detected at the plurality of second time point as the second impedance data. The first impedance data is normally distributed, and the second impedance data is normally distributed.

In some embodiments, the data distribution feature includes data discretization feature; and the determining, based on the data distribution feature of the first impedance data and the data distribution feature of the second impedance data, the first confidence level of the first impedance data and the second confidence level of the second impedance data includes: determining a first deviation value of the first impedance data based on a data discretization feature of the first impedance data; determining a second deviation value of the second impedance data based on a data discretization feature of the second impedance data; and determining the first confidence level and the second confidence level based on the first deviation value and the second deviation value.

In some embodiments, said determining the first confidence level and the second confidence level based on the first deviation value and the second deviation value includes: determining the first confidence level based on the first deviation value and the second deviation value; and determining the second confidence level based on the first confidence level and a predetermined constraint condition.

In some embodiments, the first deviation value includes a first variance of first impedance data conforming to a normal distribution, and the second deviation value includes a second variance of second impedance data conforming to a normal distribution; and the determining the first confidence level based on the first deviation value and the second deviation value includes: determining a functional relationship between a fused variance and the first confidence level based on the first variance and the second variance, where the fusion variance is a variance of fused impedance data, the fused impedance data representing a fusion relationship between the first impedance data and the second impedance data; and taking a derivative of the first confidence level based on the functional relationship between the fused variance and the first confidence level, and obtaining the first confidence level when the fusion variance is minimized.

In some embodiments, the predetermined constraint condition includes a sum of the first confidence level and the second confidence level being 1.

In some embodiments, the performing the data fusion on the first impedance data and the second impedance data based on the first confidence level and the second confidence level, to obtain the contact impedance includes: obtaining first data by multiplying the first confidence level by the first impedance data, obtaining second data by multiplying the second confidence level by the second impedance data, and adding the first data and the second data to obtain fused impedance data; determining, when the fused impedance data conforms to a normal distribution, a mean of the fused impedance data as the contact impedance.

In some embodiments, the first alternating-current constant current source applies the electrical signal to the signal acquisition electrode and a reference electrode, and the second alternating-current constant current source applies the electrical signal to the signal acquisition electrode and the reference electrode.

In a fourth aspect, provided is a human intelligence-based EEG signal validation method. The method further includes: determining, based on a power frequency interference signal that interferes with an EEG signal, first invalidity data for characterizing validity of the EEG signal; determining, based on a contact impedance, second invalidity data for characterizing validity of the EEG signal, the contact impedance including an impedance between a signal acquisition electrode and a head, and the signal acquisition electrode being configured to acquire the EEG signal; and performing data fusion on the first invalidity data and the second invalidity data, to obtain comprehensive invalidity data.

In some embodiments, the determining, based on the power frequency interference signal that interferes with the EEG signal, the first invalidity data for characterizing the validity of the EEG signal includes: determining the first invalidity data based on a signal intensity of the power frequency interference signal and a first predetermined relationship, the first predetermined relationship characterizing a relationship between the signal intensity of the power frequency interference signal and the first invalidity data.

In some embodiments, the signal intensity of the power frequency interference signal is obtained by: filtering the EEG signal based on a predetermined frequency to obtain a filtered EEG signal; obtaining the power frequency interference signal based on a difference between the EEG signal before the filtering and the filtered EEG signal; and calculating a root mean square of the power frequency interference signal as the signal intensity of the power frequency interference signal.

In some embodiments, the predetermined frequency includes 50 Hz.

In some embodiments, the first predetermined relationship includes: when the signal intensity of the power frequency interference signal is smaller than a first signal intensity threshold, a trend in which the first invalidity data increases as the signal intensity of the power frequency interference signal increases being a first trend; when the signal intensity of the power frequency interference signal is greater than or equal to the first signal intensity threshold and smaller than a second signal intensity threshold, a trend in which the first invalidity data increases as the signal intensity of the power frequency interference signal increases being a second trend; and when the signal intensity of the power frequency interference signal is greater than or equal to the second signal intensity threshold and smaller than a third signal intensity threshold, a trend in which the first invalidity data increases as the signal intensity of the power frequency interference signal increases being a third trend. The first signal intensity threshold is smaller than the second signal intensity threshold, and the second signal intensity threshold is smaller than the third signal intensity threshold. The first trend is smaller than the second trend, and the second trend is smaller than the third trend.

In some embodiments, the determining, based on the contact impedance, the second invalidity data for characterizing the validity of the EEG signal includes: determining the second invalidity data based on the contact impedance and a second predetermined relationship. The second predetermined relationship characterizes a relationship between the contact impedance and the second invalidity data.

In some embodiments, the second predetermined relationship includes: when the contact impedance is smaller than a first impedance threshold, a trend in which the second invalidity data increases as the contact impedance increases being a fourth trend; when the contact impedance is greater than or equal to the first impedance threshold and smaller than a second impedance threshold, a trend in which the second invalidity data increases as the contact impedance increases being a fifth trend; when the contact impedance is greater than or equal to the second impedance threshold and smaller than a third impedance threshold, a trend in which the second invalidity data increases as the contact impedance increases being a sixth trend. The first impedance threshold is smaller than the second impedance threshold, and the second impedance threshold is smaller than the third impedance threshold. The fourth trend is smaller than the fifth trend, and the fifth trend is smaller than the sixth trend.

In some embodiments, said performing the data fusion on the first invalidity data and the second invalidity data to obtain the comprehensive invalidity data includes: determining a first weight of the first invalidity data; determining a second weight of the second invalidity data; obtaining first data by multiplying the first invalidity data by the first weight, obtaining second data by multiplying the second invalidity data by the second weight, and adding the first data and the second data to obtain the comprehensive invalidity data.

In some embodiments, when the signal intensity of the power frequency interference signal is greater than a predetermined threshold, the first weight is greater than the second weight; when the signal intensity of the power frequency interference signal is smaller than or equal to the predetermined threshold, the first weight is smaller than or equal to the second weight; and a sum of the first weight and the second weight is 1.

In a fifth aspect, provided is a human intelligence-based impedance detection apparatus. The apparatus includes: a first detection module configured to apply, by using a first alternating-current constant current source, an electrical signal to a signal acquisition electrode, and detect first impedance data between the signal acquisition electrode and a head; a second detection module configured to apply, by using a second alternating-current constant current source, an electrical signal to the signal acquisition electrode, and detect second impedance data between the signal acquisition electrode and the head; a first determination module configured to determine, based on a data distribution feature of the first impedance data and a data distribution feature of the second impedance data, a first confidence level for the first impedance data and a second confidence level for the second impedance data; and a first fusion module configured to perform data fusion on the first impedance data and the second impedance data based on the first confidence level and the second confidence level, to obtain a contact impedance.

In a sixth aspect, provided is a human intelligence-based EEG signal verification apparatus. The apparatus further includes: a second determination module configured to determine, based on a power frequency interference signal that interferes with an EEG signal, first invalidity data for characterizing validity of the EEG signal; a third determination module configured to determine, based on a contact impedance, second invalidity data for characterizing validity of the EEG signal, the contact impedance including an impedance between a signal acquisition electrode and a head, and the signal acquisition electrode being configured to acquire the EEG signal; and a second fusion module configured to perform data fusion on the first invalidity data and the second invalidity data, to obtain comprehensive invalidity data.

In a seventh aspect, provided is a computer-readable storage medium. The medium has a computer program stored thereon. The computer program, when executed by a processor, implements the method for controlling the signal acquisition electrode according to the first aspect described above.

In an eighth aspect, provided is an electronic device. The electronic device includes: a memory; a processor; and a computer program stored in the memory and executable by the processor. The processor, when executing the computer program, implements the method for controlling the signal acquisition electrode according to the first aspect described above.

The technical solutions according to the present disclosure at least provide the following beneficial effects.

The present disclosure provides a method for controlling a signal acquisition electrode, an EEG signal acquisition apparatus, and a medium. The signal acquisition electrode is movably connected to a wearable component of the EEG signal acquisition apparatus. The wearable component is adapted to be worn on a head of a user, and the EEG signal acquisition apparatus further includes a pushing component. The method includes: controlling, when a contact impedance between the signal acquisition electrode and the head is greater than or equal to a first impedance threshold, the pushing component to push the signal acquisition electrode to move towards the head to acquire an EEG signal by using the signal acquisition electrode. The signal acquisition electrode is in full contact with the head, eliminating a need for staff to manually adjust the signal acquisition electrode, and therefore the EEG signal acquisition apparatus provided by the present disclosure has a high intelligence degree and can simplify an operation of the staff.

Additional aspects and advantages of the embodiments of present disclosure will be provided at least in part in the following description, or will become apparent in part from the following description, or can be learned from the practice of the embodiments of the present disclosure.

## BRIEF DESCRIPTION OF THE DRAWINGS

FIG. 1 is a cross-sectional view of an EEG signal acquisition apparatus according to an embodiment of the present disclosure.

FIG. 2 is a schematic structural view showing a partial structure of an EEG signal acquisition apparatus according to an embodiment of the present disclosure.

FIG. 3 is a schematic structural diagram of an EEG signal acquisition apparatus according to an embodiment of the present disclosure.

FIG. 4 is a flowchart of a method for controlling a signal acquisition electrode according to an embodiment of the present disclosure.

FIG. 5 is a schematic diagram showing a first alternating-current constant current source applying an electrical signal to a signal acquisition electrode according to an embodiment of the present disclosure.

FIG. 6 is a schematic diagram showing a second alternating-current constant current source applying an electrical signal to a signal acquisition electrode according to an embodiment of the present disclosure.

FIG. 7 is a graph of a first predetermined relationship according to an embodiment of the present disclosure.

FIG. 8 is a graph of a second predetermined relationship according to an embodiment of the present disclosure.

FIG. 9 is a graph of a first weight and a second weight according to an embodiment of the present disclosure.

FIG. 10 is a schematic flowchart of a human intelligence-based impedance detection method according to an embodiment of the present disclosure.

FIG. 11 is a schematic flowchart of a human intelligence-based EEG signal verification method according to an embodiment of the present disclosure.

FIG. 12 is a schematic diagram of a human intelligence-based impedance detection apparatus according to an embodiment of the present disclosure.

FIG. 13 is a schematic diagram of a human intelligence-based EEG signal verification apparatus according to an embodiment of the present disclosure.

FIG. 14 is a schematic structural diagram of an electronic device according to an embodiment of the present disclosure.

## DETAILED DESCRIPTION OF THE EMBODIMENTS

Embodiments of the present disclosure are described in detail below, examples of which are illustrated in the accompanying drawings, where the same or similar reference numerals throughout represent the same or similar elements or elements with the same or similar functions. The embodiments described below with reference to the accompanying drawings are illustrative only, and are intended to explain rather than limit the present disclosure.

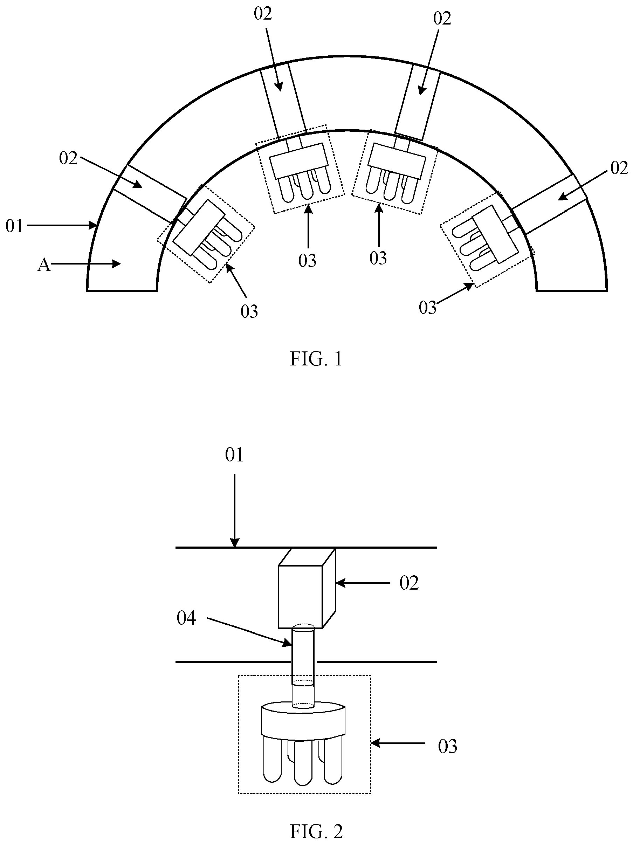

Embodiments of the present disclosure provide an EEG signal acquisition apparatus. Referring to FIG. 1, the apparatus includes a wearable component 01, a pushing component 02, and a signal acquisition electrode 03. It can be seen from FIG. 1 that the apparatus may include a plurality of pushing components 02 and a plurality of signal acquisition electrodes 03. The plurality of signal acquisition electrodes 03 and the plurality of pushing components 02 may be in one-to-one correspondence.

The wearable component 01 is adapted to be worn on a head of a user and has a mounting cavity A. The pushing component 02 is located in the mounting cavity A. The signal acquisition electrode 03 is movably connected to the wearable component 01. For example, the signal acquisition electrode 03 may be movably connected to an inner side of the wearable component 01 (i.e., a side close to the head of the user).

After the user wears the wearable component 01 on the head of the user, the pushing component 02 is configured to push the signal acquisition electrode 03 to move towards the head when contact impedance between the signal acquisition electrode 03 and the head is greater than or equal to a first impedance threshold, to automatically adjust the contact impedance between the signal acquisition electrode 03 and the head. The contact impedance between the signal acquisition electrode 03 and the head refers to a contact impedance between the signal acquisition electrode 03 and a skin of the head.

It can be understood that the pushing component 02 may push the signal acquisition electrode 03 a plurality of times until the contact impedance is smaller than the first impedance threshold. The first impedance threshold refers to a maximum allowable contact impedance between the signal acquisition electrode 03 and the head of the user that causes the signal quality of the EEG signal acquired by the EEG signal acquisition apparatus to satisfy a quality requirement. That is, the first impedance threshold is a maximum contact impedance that does not affect the quality of the EEG signal acquisition.

In summary, the embodiments of the present disclosure provide the EEG signal acquisition apparatus, which includes the wearable component, the signal acquisition electrode movably connected to the wearable component, and the pushing component located in the mounting cavity of the wearable component. The pushing component can automatically push the signal acquisition electrode to move towards the head of the user when the contact impedance between the signal acquisition electrode and the head of the user is greater than or equal to the first impedance threshold, to allow the signal acquisition electrode to be in full contact with the head. The signal acquisition electrode is in full contact with the head, eliminating a need for staff to manually adjust the signal acquisition electrode. Therefore the EEG signal acquisition apparatus according to the embodiments of the present disclosure has a high intelligence degree and can simplify an operation of staff.

In an embodiment of the present disclosure, referring to FIG. 2, the EEG signal acquisition apparatus may further include a conductive rod 04. The mounting cavity A of the wearable component 01 is provided with a through hole (not shown in FIG. 2) at a side of the mounting cavity A. The conductive rod 04 may be located in the through hole to be movably connected to the wearable component 01. The conductive rod 04 has an end connected to the signal acquisition electrode 03 and another end that is configured to abut with the pushing component 02.

The pushing component 02 may push the conductive rod 04 to move towards the head of the user when the contact impedance between the signal acquisition electrode 03 and the head of the user is greater than or equal to the first impedance threshold. The conductive rod 04 may then bring the signal acquisition electrode 03 to move towards the head of the user.

In an embodiment of the present disclosure, the EEG signal acquisition apparatus may be connected to an electronic device (such as a computer). After the user wears the wearable component 01 on the head of the user, the electronic device may obtain the contact impedance between the signal acquisition electrode 03 and the head, and may control the pushing component 02 to automatically push the signal acquisition electrode 03 to move towards the head when it is determined that the contact impedance is greater than or equal to the first impedance threshold. The electronic device may be independent of the EEG signal acquisition apparatus and may pre-store the first impedance threshold.

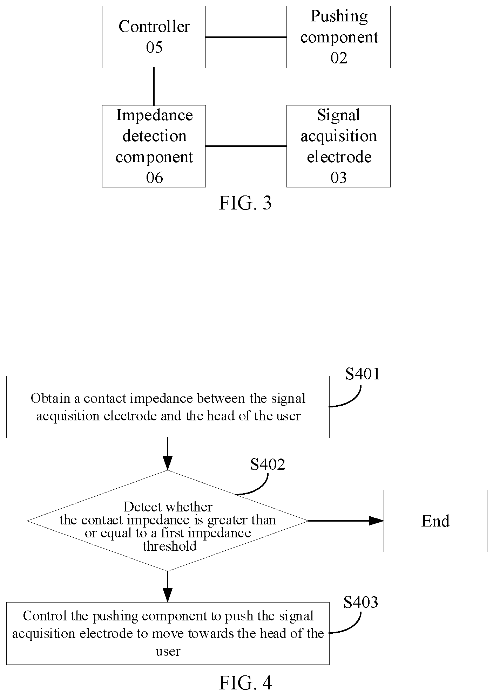

In an embodiment of the present disclosure, referring to FIG. 3, the EEG signal acquisition apparatus may further include a controller 05, which may be connected to the pushing component 02. After the user wears the wearable component 01 on the head of the user, the controller 05 may obtain the contact impedance between the signal acquisition electrode 03 and the head. When it is determined by the controller 05 that the contact impedance is greater than or equal to the first impedance threshold, it may be determined that the signal acquisition electrode 03 is not in sufficient contact with the skin of the head, and then may control the pushing component 02 to push the signal acquisition electrode 03 to move towards the head, to allow the signal acquisition electrode 03 to be in sufficient contact with the head. The controller 05 may pre-store the first impedance threshold.

In an embodiment of the present disclosure, the pushing component 02 may be implemented in various manners. The following exemplary implementations are taken as an example to illustrate the pushing component 02, in the embodiments of the present disclosure.

In a first exemplary implementation, the pushing component 02 may be an airbag. The airbag has an end that may be fixedly connected to an inner wall of the mounting cavity A of the wearable component 01, for example, through bonding. The airbag has another end that abuts against the signal acquisition electrode 03 after inflation. The airbag is configured to be inflated when the contact impedance between the signal acquisition electrode 03 and the head is greater than or equal to the first impedance threshold, to push the signal acquisition electrode 03 to move towards the head.

For the first implementation, the EEG signal acquisition apparatus may further include an inflation component connected to the airbag. The inflation component is configured to inflate the airbag when the contact impedance between the signal acquisition electrode 03 and the head is greater than or equal to the first impedance threshold. In an exemplary embodiment of the present disclosure, the inflation component may be a blower or an air pump.

In an embodiment of the present disclosure, the EEG signal acquisition apparatus may further include an air duct. The air duct has an end that may be connected to the airbag and another end that may be connected to the inflation component. In this way, the inflation component may inflate the airbag through the air duct.

It can be understood that the inflation component may be connected to the controller 05 of the EEG signal acquisition apparatus. The controller 05 may be configured to control the inflation component to inflate the airbag when the contact impedance between the signal acquisition electrode 03 and the head is greater than or equal to the first impedance threshold. In an embodiment of the present disclosure, the inflation component may be connected to an electronic device. The electronic device may be configured to control the inflation component to inflate the airbag when the contact impedance between the signal acquisition electrode 03 and the head is greater than or equal to the first impedance threshold.

It can be understood that the mounting cavity A of the wearable component 01 may have a storage groove at an inner wall of the mounting cavity A. The airbag may be disposed in the storage groove. For example, the airbag may be bonded to a groove wall of the storage groove. The airbag may be located in the storage groove when not inflated to allow for storage of the airbag. After the airbag is inflated, the airbag may be partially located outside the storage groove.

In a second exemplary implementation, the pushing component 02 may be a retractable component, such as a retractable rod. The retractable component has an end that may be connected to the inner wall of the mounting cavity A of the wearable component 01 and another end that may abut against the signal acquisition electrode 03 when the retractable component is in an extended state. The retractable component may be configured to be in the extended state when the contact impedance between the signal acquisition electrode and the head is greater than or equal to the first impedance threshold, to push the signal acquisition electrode 03 to move towards the head of the user.

In a third exemplary implementation, the pushing component 02 may be an electromagnet connected to the signal acquisition electrode 03. In this case, the mounting cavity A of the wearable component 01 may be further provided with a magnet at the inner wall of the mounting cavity A, and the magnet faces towards the pushing component 02. For example, an orthographic projection of the magnet on a plane where the pushing component 02 is located coincides with the pushing component 02, or is located in the pushing component 02. The magnet may be a permanent magnet or an electromagnet.

The pushing component 02 may be configured to be energized when the contact impedance between the signal acquisition electrode and the head is greater than or equal to the first impedance threshold, to generate a magnetic field opposite to that of the magnet, thereby pushing the signal acquisition electrode 03 to move towards the head of the user.

For the third optional implementation, the EEG signal acquisition apparatus may further include a power supply component. The power supply component may be connected to the pushing component 02 and may be configured to provide an electrical signal to the pushing component 02 to cause the pushing component 02 to generate a magnetic field opposite to that of the mounting component.

For example, the power supply component may provide an electrical signal to the pushing component 02 under a control of the controller 05 of the EEG signal acquisition apparatus or the electronic device. The electrical signal may be a current signal or a voltage signal.

In an embodiment of the present disclosure, referring to FIG. 3, the EEG signal acquisition apparatus may further include an impedance detection component 06. As shown in FIG. 3, the impedance detection component 06 may be connected to the signal acquisition electrode 03 and the controller 05. After the EEG signal acquisition apparatus is worn on the head of the user and an impedance measuring process is started, the impedance detection component 06 may detect the contact impedance between the signal acquisition electrode 03 and the head of the user, and may send the contact impedance to the controller 05. Correspondingly, the controller 05 may obtain the contact impedance.

In an exemplary embodiment of the present disclosure, the pushing component 02 may be configured to push the signal acquisition electrode 03 in accordance with a reference intensity corresponding to the contact impedance between the signal acquisition electrode 03 and the head. The reference intensity is determined based on the contact impedance and a correspondence between impedances and intensities. A plurality of impedances are recorded in the correspondence, and any two of the plurality of impedances differ from each other. Any two of the intensities also differ from each other.

It can be seen that the pushing component 02 may push the signal acquisition electrode 03 with different intensities. In this way, control flexibility of the signal acquisition electrode 03 is improved.

It can be understood that the intensity recorded in the correspondence may increase as the impedance increases, that is, the intensity recorded in the correspondence is positively correlated with the impedance. In this way, the signal acquisition electrode may be pushed with a greater pushing intensity when the contact impedance is greater, such that the signal acquisition electrode 03 may be in close contacted with the head quickly, which shortens a time period taken for the contact impedance between the signal acquisition electrode and the head to be reduced below the first impedance threshold, and improves control efficiency of the signal acquisition electrode.

In an embodiment of the present disclosure, the controller 05 (or the electronic device) may pre-store the correspondence between impedances and intensities. After the controller 05 (or electronic device) obtains the contact impedance between the signal acquisition electrode and the head of the user, a reference pushing intensity corresponding to the contact impedance may be determined from the correspondence.

It can be understood that if the pushing component 02 is an airbag, the intensity recorded in the correspondence between the impedances and the intensities may refer to an amount of air inflated by the inflation component for inflating the airbag each time. If the pushing component 02 is a retractable component, the intensity recorded in the correspondence between the impedances and the intensities may refer to an extending length by which the retractable component is extended each time. If the pushing component 02 is an electromagnet, the intensity recorded in the correspondence between the impedances and the intensities may refer to magnitude of the electrical signal provided by the power supply component to the electromagnet each time.

In an embodiment of the present disclosure, if it is determined by the controller 05 (or the electronic device) that a contact impedance of each of a target number of signal acquisition electrodes 03 among the plurality of signal acquisition electrodes 03 of the EEG signal acquisition apparatus satisfies a target condition, the EEG signal may be acquired by the plurality of signal acquisition electrodes 03. The target number is smaller than or equal to a total number of the plurality of signal acquisition electrodes 03 and is greater than a number threshold. The target condition includes a duration during which the contact impedance is smaller than the first impedance threshold reaching a target duration. Both the number threshold and the target duration may be pre-stored by the controller 05. For example, the number threshold may be four-fifths of the total number. The target duration may be 10 minutes.

For example, the target number may be the total number of the plurality of signal acquisition electrodes. That is, after it is determined by the controller 05 (or the electronic device) that contact impedances of all the signal acquisition electrodes 03 of the EEG signal acquisition apparatus satisfy the target condition, an EEG signal of the user may be acquired. That is, the controller 05 starts acquiring the EEG signal only after it is determined that the signal acquisition electrode 03 is stable relative to the head. In this way, noises in the acquired EEG signal can be reduced, thereby ensuring better quality of the acquired EEG signal.

According to the above description, the EEG signal acquisition apparatus according to the embodiment of the present disclosure can realize automatic adjustment of the contact impedance, and can automatically start acquiring the EEG signal after the contact impedances of the target number of signal acquisition electrodes 03 satisfy the target condition.

In an embodiment of the present disclosure, the signal acquisition electrode 03 may be a needle-shaped electrode. The wearable component 01 may be of a cap shape, or may be of a ring shape. When the wearable component 01 is in the ring shape, the EEG signal acquisition apparatus may further include a plurality of sheet-shaped electrodes. The plurality of sheet-shaped electrodes and a plurality of needle-shaped electrodes may be arranged opposite to each other. For example, 5 sheet-shaped electrodes may be provided, and 4 needle-shaped electrodes may be provided. One of the 5 sheet-shaped electrodes is a ground (GND) electrode. That is, the EEG signal acquisition apparatus is an 8-channel EEG signal acquisition apparatus.

In a process of acquiring the EEG signal of the user, the plurality of sheet-shaped electrodes may be in contact with the forehead of the user, and the plurality of needle-shaped electrodes may be in contact with the back of the head of the user.

In an exemplary embodiment of the present disclosure, the wearable component 01 may be made of a flexible material.

In summary, the embodiments of the present disclosure provide the EEG signal acquisition apparatus, which includes the wearable component, the signal acquisition electrode movably connected to the wearable component, and the pushing component located in the mounting cavity of the wearable component. The pushing component may automatically push the signal acquisition electrode to move towards the head of the user when the contact impedance between the signal acquisition electrode and the head of the user is greater than or equal to the first impedance threshold, to allow the signal acquisition electrode to be in full contact with the head. The signal acquisition electrode is in full contact with the head, eliminating a need for staff to manually adjust the signal acquisition electrode. Therefore the EEG signal acquisition apparatus provided by the present disclosure has a high intelligence degree and can simplify the operation of the staff.

Embodiments of the present disclosure provide a method for controlling a signal acquisition electrode, which is applied in a controller of an EEG signal acquisition apparatus or the electronic device described above. The EEG signal acquisition apparatus further includes a wearable component and a pushing component. The signal acquisition electrode is movably connected to the wearable component, and the pushing component may push the signal acquisition electrode.

The method for controlling the signal acquisition electrode according to the embodiments of the present disclosure will be exemplarily described by taking the controller of the EEG signal acquisition apparatus controlling the pushing component as an example. Referring to FIG. 4, the method includes actions at steps S401 to S403.

At step S401, a contact impedance between the signal acquisition electrode and the head of the user is obtained.

In an embodiment of the present disclosure, the EEG signal acquisition apparatus may include an impedance detection component that may be connected to the signal acquisition electrode and the controller. After the EEG signal acquisition apparatus is worn on the head of the user, the impedance detection component may detect the contact impedance between the signal acquisition electrode and the head of the user, and may send the contact impedance to the controller. Correspondingly, the controller may obtain the contact impedance between the signal acquisition electrode and the head.

It can be understood that the impedance detection component may be connected to each of a plurality of signal acquisition electrodes, and may determine a contact impedance between each of the plurality of signal acquisition electrodes and the head of the user.

At step S402, whether the contact impedance is greater than or equal to a first impedance threshold is detected.

The first impedance threshold may be pre-stored by the controller and is a maximum contact impedance that does not affect quality of the EEG signal acquisition.

After the controller obtains the contact impedance between the signal acquisition electrode and the head of the user, whether the contact impedance is greater than the first impedance threshold may be detected. If it is determined by the controller that the contact impedance between the signal acquisition electrode and the head of the user is greater than or equal to the first impedance threshold, step S403 may be performed. If it is determined by the controller that the contact impedance between the signal acquisition electrode and the head of the user is smaller than the first impedance threshold, it may be determined that the contact impedance satisfies an acquisition requirement of the EEG signal, and then an operation of pushing the signal acquisition electrode by controlling the pushing component may be ended.

At step S403, the pushing component is controlled to push the signal acquisition electrode to move towards the head of the user.

If it is determined by the controller that the contact impedance is greater than or equal to the first impedance threshold, the pushing component may push the signal acquisition electrode to move towards the head of the user to acquire the EEG signal through the signal acquisition electrode.

For example, the controller may control the pushing component to push the signal acquisition electrode to move towards the head a plurality of times until the contact impedance is smaller than the first impedance threshold.

In an exemplary implementation, the controller has a correspondence between impedances and intensities pre-stored therein. A plurality of impedances are recorded in the correspondence, and any two of the plurality of impedances differ from each other. After it is determined by the controller that the contact impedance between the signal acquisition electrode and the head of the user is greater than or equal to the first impedance threshold, a reference pushing intensity corresponding to the contact impedance may be determined from the correspondence between the impedances and the intensities. Then, the controller may control the pushing component to push the signal acquisition electrode towards the head in accordance with the reference pushing intensity. In this way, an effect of pushing the signal acquisition electrode with different intensities can be achieved.

It can be understood that in this implementation, the pushing component may push the signal acquisition electrode in accordance with the reference pushing intensity each time.

In another exemplary implementation, after it is determined by the controller that the contact impedance between the signal acquisition electrode and the head of the user is greater than or equal to the first impedance threshold, a target pushing intensity of the pushing component may be determined based on a difference between the first impedance threshold and the contact impedance. Then, the controller may control the pushing component to push the signal acquisition electrode to move towards the head in accordance with the target pushing intensity. The target pushing intensity may be positively correlated with the difference.

It can be understood that the controller may directly determine the target pushing intensity of the pushing component based on the difference. In an embodiment of the present disclosure, the controller may process the difference between the first impedance threshold and the contact impedance with a proportional-integral-derivative (PID) algorithm to obtain the target pushing intensity of the pushing component.

Since the target pushing intensity is obtained by the controller processing the difference with the PID algorithm, by the pushing component pushing the signal acquisition component in accordance with the target pushing intensity, the contact impedance between the signal acquisition component and the head of the user can be quickly and smoothly reduced below the first impedance threshold.

In an embodiment of the present disclosure, if it is determined by the controller that a contact impedance of each of a target number of signal acquisition electrodes among the plurality of signal acquisition electrodes of the EEG signal acquisition apparatus satisfies a target condition, the EEG signal may be acquired by the plurality of signal acquisition electrodes.

For example, after it is determined by the controller that contact impedances of all the signal acquisition electrodes of the EEG signal acquisition apparatus satisfy the target condition, The EEG signal of the user may be acquired by the plurality of signal acquisition electrodes. In this way, better quality of the acquired EEG signal can be ensured.

It can be understood that the sequence of the steps of the method for controlling the signal acquisition electrode according to the embodiments of the present disclosure can be appropriately adjusted, and the steps can also be increased or decreased accordingly. Various variants and alternatives that can be easily conceived by any of those skilled in the art within the technical scope of the present disclosure shall fall within the scope of protection of the present disclosure, and therefore details thereof will be omitted.

In summary, the embodiments of the present disclosure provide the EEG signal acquisition method, with which the signal acquisition electrode may be automatically controlled and pushed to move towards the head of the user when the contact impedance between the signal acquisition electrode and the head of the user is greater than or equal to the first impedance threshold, to allow the signal acquisition electrode to be in full contact with the head. The signal acquisition electrode is in full contact with the head, eliminating a need for staff to manually adjust the signal acquisition electrode, and therefore the method has a high intelligence degree and can simplify an operation of the staff.

In another example, the method for controlling the signal acquisition electrode may further include: detecting the contact impedance, for example, by using an impedance detection component. The detecting the contact impedance may be performed between step S401 and step S402 shown in FIG. 4, for example. The detecting the contact impedance includes: applying, by using a first alternating-current constant current source, an electrical signal to the signal acquisition electrode, and detecting first impedance data between the signal acquisition electrode and the head; applying, by using a second alternating-current constant current source, an electrical signal to the signal acquisition electrode, and detecting second impedance data between the signal acquisition electrode and the head; determining, based on a data distribution characteristic of the first impedance data and a data distribution characteristic of the second impedance data, a first confidence level for the first impedance data and a second confidence level for the second impedance data; and performing data fusion on the first impedance data and the second impedance data based on the first confidence level and the second confidence level, to obtain the contact impedance.

In an embodiment, the first impedance data between the signal acquisition electrode and the head includes first impedance data between the signal acquisition electrode and a scalp. The first alternating-current constant current source differs from the second alternating-current constant current source. Both the first alternating-current constant current source and the second alternating-current constant current source may be used to apply a constant current electrical signal. In an embodiment, a frequency of the electrical signal applied by the first alternating-current constant current source differs from a frequency of the electrical signal applied by the second alternating-current constant current source. For example, the frequency of the electrical signal applied by the first alternating-current constant current source is greater than the frequency of the electrical signal applied by the second alternating-current constant current source. For example, the frequency of the electrical signal applied by the first alternating-current constant current source may be about 80 Hz, and the frequency of the electrical signal applied by the second alternating-current constant current source may be about 10 Hz, about 20 Hz, about 30 Hz, about 40 Hz, etc.

In an embodiment, the first alternating-current constant current source is provided by an integrated chip for measuring the EEG signal. That is, the first alternating-current constant current source is directly provided by the integrated chip for measuring the EEG signal rather than being provided additionally. In this way, cost of impedance detection apparatus is reduced.

In an embodiment, the second alternating-current constant current source may be provided by a Wien bridge self-excited oscillation circuit. For example, the Wien bridge self-excited oscillation circuit may generate a sinusoidal wave signal that may be applied to the signal acquisition electrode.

In an embodiment, the first alternating-current constant current source applies an electrical signal to the signal acquisition electrode and a reference electrode, and the second alternating-current constant current source applies an electrical signal to the signal acquisition electrode and the reference electrode. The reference electrode is generally configured to determine the signal reference.

After the first impedance data and the second impedance data are detected, the first confidence level of the first impedance data and the second confidence level of the second impedance data are determined based on a data distribution feature of the first impedance data and a data distribution feature of the second impedance data. Concentrated impedance data distribution with lower dispersion degree correspond to a higher confidence level.

After the first confidence level and the second confidence level are obtained, the first impedance data and the second impedance data may be fused based on the first confidence level and the second confidence level to obtain the contact impedance. Compared with first impedance data and second impedance data obtained by a single alternating-current constant current source, the contact impedance more accurately reflects the impedance between the signal acquisition electrode and the head. It can be seen that the impedance detection method of the present disclosure can improve accuracy of the impedance detection.

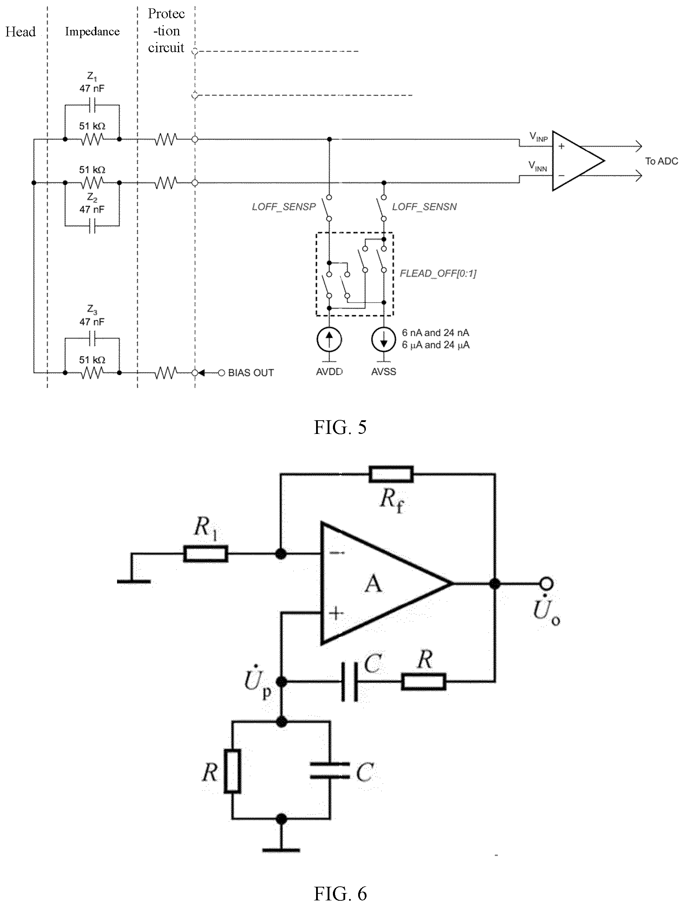

FIG. 5 is a schematic diagram showing a first alternating-current constant current source applying an electrical signal to a signal acquisition electrode according to an embodiment of the present disclosure.

As shown in FIG. 5, the first alternating-current constant current source is provided by an integrated chip. In FIG. 5, DVSS represents Digital VSS, which is ground of a digital part of a circuit; AVDD represents an analog voltage, which powers an analog device in the chip; each of LOFF_SENSP, LOFF_SENSN, and FLEAD_OFF represents a register; ADC represents an analog-to-digital converter; BIAS OUT pin is provided with a bias current. A chip protection circuit prevents an excessive current through a resistor.

$Z_1$, $Z_2$, and $Z_3$ represent impedances between the signal acquisition electrode and the head (the scalp). Resistance exists when the signal acquisition electrode is in contact with the scalp, and therefore $Z_1$, $Z_2$, and $Z_3$ represent impedances.

FIG. 6 is a schematic diagram showing a second alternating-current constant current source applying an electrical signal to a signal acquisition electrode according to an embodiment of the present disclosure.

As shown in FIG. 6, the second alternating-current constant current source may be provided by a Wien bridge self-excited oscillation circuit. FIG. 6 shows an example of the Wien bridge self-excited oscillation circuit for providing a sinusoidal wave signal and applying the sinusoidal wave signal to the signal acquisition electrode and the reference electrode.

In an embodiment, impedance data is detected at a plurality of first time points, and impedance data detected at adjacent time points are selected from the impedance data detected at the plurality of first time points as the first impedance data. For example, 100 pieces of impedance data are detected at 100 time points, and 10 pieces of impedance data corresponding to last 10 time points are selected as the first impedance data. The time points corresponding to the last 10 pieces of impedance data are most recent time points. Since the impedance changes over time, the 10 pieces of impedance data at the adjacent time points has better timeliness. Similarly, impedance data is detected at a plurality of second time points, and impedance data detected at adjacent time points are selected from the impedance data detected at the plurality of second time point as the second impedance data. For example, the plurality of second time points also include 100 time points, and impedance data corresponding to 10 adjacent time points are selected as the second impedance data. Both the first impedance data and the second impedance data are normally distributed.

It can be understood that the accuracy of the impedance detection is improved by selecting the impedance data with the better timeliness for subsequent processing.

In an embodiment, the data distribution feature includes a data discretization feature. A first deviation value of the first impedance data may be determined based on a data discretization feature of the first impedance data; a second deviation value of the second impedance data may be determined based on a data discretization feature of the second impedance data; and the first confidence level and the second confidence level may be determined based on the first deviation value and the second deviation value.

In an embodiment, the first confidence level is determined based on the first deviation value and the second deviation value; and the second confidence level is determined based on the first confidence level and a predetermined constraint condition. The predetermined constraint condition includes a sum of the first confidence level and the second confidence level being 1.

In an embodiment, as shown in formula (1), the first impedance data is represented as $x_1$, a first deviation value of first impedance data $x_1$ conforming to a normal distribution includes a first variance $\sigma_1^2$, $\mu_1$ is a mean of the first impedance data $x_1$, and the first impedance data $x_1$ is estimated by maximum likelihood to obtain the mean $\mu_1$ and the first variance $\sigma_1^2$.

$$\mu_1 = \overline{x_1}, \qquad \sigma_1^2 = \frac{1}{n}\sum_{i=1}^{n}\left(x_{1_i} - \mu_1\right)^2 \quad (1)$$

where n represents the number of data in the first impedance data $x_1$. As described above, when the first impedance data $x_1$ includes 10 pieces of impedance data, n=10.

As shown in formula (2), the second impedance data is represented as $x_2$, a second deviation value of the second impedance data $x_2$ conforming to a normal distribution includes a second variance $\sigma_2^2$, $\mu_2$ is a mean of the second impedance data $x_2$, and the second impedance data $x_2$ is estimated by maximum likelihood to obtain the mean $\mu_2$ and the first variance $\sigma_2^2$.

$$\mu_2 = \overline{x_2}, \qquad \sigma_2^2 = \frac{1}{n}\sum_{i=1}^{n}\left(x_{2_i} - \mu_2\right)^2 \quad (2)$$

where n represents the number of data in the second impedance data $x_2$. As mentioned above, when the sec-

ond impedance data $x_2$ includes 10 pieces of impedance data, n=10.

As shown in formula (3), $\alpha$ represents a first confidence level; $\beta$ represents a second confidence level; and a sum of the first confidence level $\alpha$ and the second confidence level $\beta$ is 1. Fused impedance data $\hat{x}$ represents a fusion relationship between the first impedance data $x_1$ and the second impedance data $x_2$. The fused impedance data $\hat{x}$ is obtained by fusing the first impedance data $x_1$ and the second impedance data $x_2$.

$$\hat{x} = \alpha x_1 + \beta x_2$$
$$\alpha + \beta = 1 \qquad (3)$$
$$\Rightarrow \hat{x} = \alpha x_1 + (1-\alpha)x_2$$

After the first variance $\sigma_1^2$ and the second variance $\sigma_2^2$ are obtained, a functional relationship between fused variance $\hat{\sigma}^2$ and the first confidence level a may be determined based on the first variance $\sigma_1^2$ and the second variance $\sigma_2^2$, which is shown in formula (4). The fused variance $\hat{\sigma}^2$ is a variance of the fused impedance data $\hat{x}$.

$$\hat{\sigma}^2(\alpha) = (1-\alpha)^2\sigma_1^2 + \alpha^2\sigma_2^2 \quad (4)$$

A derivative of the first confidence level $\alpha$ is taken based on the functional relationship between the fused variance $\hat{\sigma}^2$ and the first confidence level a, and the first confidence level a is obtained when the fused variance $\hat{\sigma}^2$ is minimized, as shown in formula (5).

$$\alpha = \frac{\sigma_1^2}{\sigma_1^2 + \sigma_2^2} \quad (5)$$

The second confidence level $\beta$ is obtained based on a constraint relationship between the first confidence level $\alpha$ and the second confidence level $\beta$, as shown in formula (6).

$$\beta = \frac{\sigma_2^2}{\sigma_1^2 + \sigma_2^2} \quad (6)$$

As shown in formula (7), the first data is obtained by multiplying the first confidence level a by the first impedance data $x_1$, the second data is obtained by multiplying the second confidence level $\beta$ by the second impedance data $x_2$, and the first data and the second data are added to obtain the fused impedance data $\hat{x}$. When the fused impedance data $\hat{x}$ conforms to the normal distribution, a mean of the fused impedance data $x_2$ is

determined as a final contact impedance.

$$\hat{x}(x_1, x_2) = \frac{\sigma_1^2}{\sigma_1^2 + \sigma_2^2} x_1 + \frac{\sigma_2^2}{\sigma_1^2 + \sigma_2^2} x_2 \quad (7)$$

In another embodiment, the method for controlling the signal acquisition electrode may further include: subsequent to the EEG signal having been acquired by the signal acquisition electrode, verifying the EEG signal. For example, the EEG signal may be verified by an impedance detection component or a controller. As shown in FIG. 4, after the step S403 is performed, the EEG signal is acquired by the signal acquisition electrode and then is verified. The verifying the EEG signal includes: determining, based on a power frequency interference signal that interferes with the EEG signal, first invalidity data for characterizing validity of the EEG signal; determining, based on the contact impedance, second invalidity data for characterizing validity of the EEG signal; and performing data fusion on the first invalidity data and the second invalidity data, to obtain comprehensive invalidity data.

The power frequency interference signal includes, for example, an environmental noise signal. In the process of acquiring the EEG signal, the environmental noise may interfere with the acquisition process, resulting in fusion of the power frequency interference signal into the acquired EEG signal, which reduces validity of the EEG signal. Therefore, the power frequency interference signal may be separated from the acquired EEG signal, and the first invalidity data characterizing the validity of the EEG signal may be determined based on the power frequency interference signal.

In an embodiment, the contact impedance includes an impedance between the signal acquisition electrode and the head, and the signal acquisition electrode is configured to acquire the EEG signal. The impedance between the signal acquisition electrode and the head includes, for example, the impedance between the signal acquisition electrode and the head scalp. When the EEG signal is acquired, the signal acquisition electrode is attached to the scalp. In this case, an impedance is formed between the signal acquisition electrode and the scalp. The contact impedance may be, for example, an impedance value. A greater impedance indicates a worse contact between the signal acquisition electrode and the scalp, and lower quality of the acquired EEG signal. Therefore, the second invalidity data characterizing the validity of the EEG signal may be determined based on the contact impedance.

In an embodiment, the first invalidity data and the second invalidity data that characterize the validity of the EEG signal are obtained in different manners, such that obtained fused invalidity data can more comprehensively and accurately represent the validity of the EEG signal. It can be seen that the validity of the EEG signal is comprehensively verified based on the power frequency interference signal and the contact impedance, to achieve comprehensive verification from multiple perspective. In this way, verification accuracy and verification effect are improved.

In another embodiment, the first invalidity data may be determined based on a signal intensity of the power frequency interference signal and a first predetermined relationship. The first predetermined relationship characterizes a relationship between the signal intensity of the power frequency interference signal and the first invalidity data.

In an embodiment, the EEG signal may be filtered based on a predetermined frequency to obtain a filtered EEG signal. A frequency of the environmental noise interference signal is typically around 50 Hz. Therefore, the predetermined frequency used in the filtering includes 50 Hz, or may be around 50 Hz, such as 40 Hz to 60 Hz. A recursive filter (IIR digital filter) may be used for the filtering.

After the filtering, the power frequency interference signal is obtained based on a difference between the EEG signal before the filtering and the filtered EEG signal; and a root mean square of the power frequency interference signal is calculated as the signal intensity of the power frequency interference signal. Greater signal intensity of the power frequency interference signal indicates a higher degree of the power frequency interference during the EEG signal acquisition. In this case, the first invalidity data indicates a higher degree of invalidity of the EEG signal.

In an embodiment, when the signal intensity of the power frequency interference signal is smaller than a first signal intensity threshold $K_1$, a trend in which the first invalidity data increases as the signal intensity of the power frequency interference signal increases is a first trend. In an example, the first trend represents that the first invalidity data increases slowly or remains unchanged as the signal intensity of the power frequency interference signal increases, indicating that when the signal intensity of the power frequency interference signal is smaller than first signal intensity threshold $K_1$, the power frequency interference signal has a relatively small impact on the EEG signal. In this case, a degree of validity of the EEG signal is relatively high.

When the signal intensity of the power frequency interference signal is greater than or equal to the first signal intensity threshold $K_1$ and smaller than a second signal intensity threshold $K_2$, a trend in which the first invalidity data increases as the signal intensity of the power frequency interference signal increases is a second trend. In an example, the second trend represents that the first invalidity data increases more rapidly as the signal intensity of the power frequency interference signal increases, indicating that when the signal intensity of the power frequency interference signal is greater than or equal to the first signal intensity threshold $K_1$ and smaller than the second signal intensity threshold $K_2$, the power frequency interference signal has a relatively large impact on the EEG signal. In this case, the degree of validity of the EEG signal is relatively low.

When the signal intensity of the power frequency interference signal is greater than or equal to the second signal intensity threshold $K_2$ and smaller than a third signal intensity threshold $K_3$, a trend in which the first invalidity data increases as the signal intensity of the power frequency interference signal increases is a third trend. In an example, the third trend, for example, indicates that the first invalidity data increases more rapidly as the signal intensity of the power frequency interference signal increases, indicating that when the signal intensity of the power frequency interference signal is greater than or equal to second signal intensity threshold $K_2$ and smaller than the third signal intensity threshold $K_3$, the power frequency interference signal has a huge impact on the EEG signal. In this case, the degree of validity of the EEG signal is very low.

The first signal intensity threshold $K_1$ is smaller than the second signal intensity threshold $K_2$, and the second signal intensity threshold $K_2$ is smaller than the third signal intensity threshold $K_3$. The first trend is smaller than the second trend, and the second trend is smaller than the third trend.

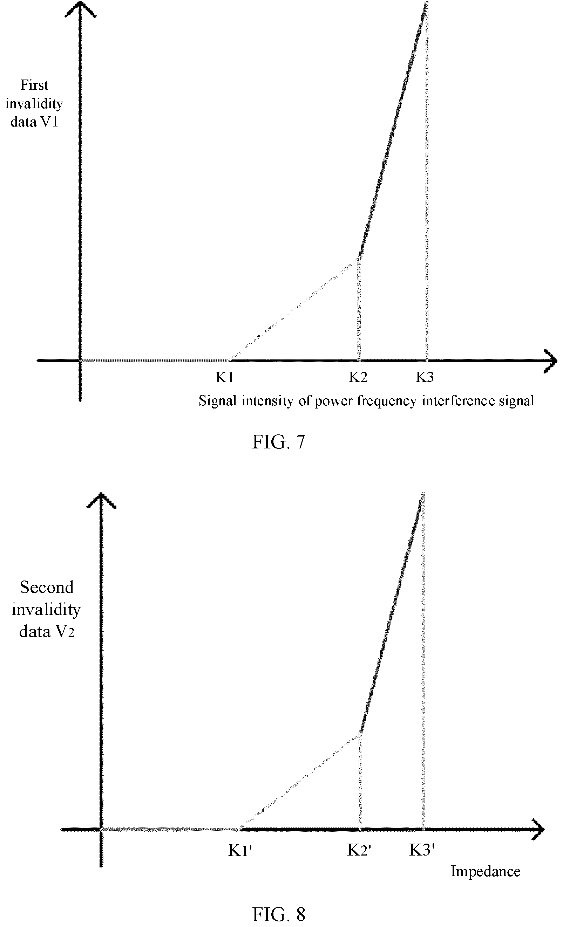

FIG. 7 is a graph of a first predetermined relationship according to an embodiment of the present disclosure.

As shown in FIG. 7, the first predetermined relationship may include, for example, a functional relationship between the signal intensity of the power frequency interference signal and the first invalidity data $V_1$, which is, for example, a piecewise function. FIG. 7 shows that the piecewise function has three intervals.

In a first interval of the function, the signal intensity of the power frequency interference signal is smaller than the first signal intensity threshold $K_1$, and the first invalidity data $V_1$ remains unchanged as the signal intensity of the power frequency interference signal increases, indicating that the power frequency interference signal has a relatively small impact on the EEG signal. In this case, the degree of validity of the EEG signal is relatively high.

In a second interval of the function, the signal intensity of the power frequency interference signal is greater than or equal to the first signal intensity threshold $K_1$ and smaller than the second signal intensity threshold $K_2$, and the first invalidity data $V_1$ increases rapidly as the signal intensity of the power frequency interference signal increases, indicating that the power frequency interference signal has a relatively large impact on the EEG signal. In this case, the degree of validity of the EEG signal is relatively low.

In a third interval of the function, the signal intensity of the power frequency interference signal is greater than or equal to the second signal intensity threshold $K_2$ and smaller than the third signal intensity threshold $K_3$, and the first invalidity data $V_1$ increases more rapidly as the signal intensity of the power frequency interference signal increases, indicating that the power frequency interference signal has a huge impact on the EEG signal. In this case, the degree of validity of the EEG signal is very low.

It can be understood that in the embodiments of the present disclosure, the first invalidity data is determined based on the different signal intensity thresholds and distinguished based on the different situations. In this way, accuracy of the first invalidity data is improved. Therefore, the verification effect of EEG signal is improved.

In another embodiment, the second invalidity data is determined based on the contact impedance and a second predetermined relationship. The second predetermined relationship characterizes a relationship between the contact impedance and the second invalidity data.

In an embodiment, when the contact impedance is smaller than a first impedance threshold $K_1'$, a trend in which the second invalidity data increases as the contact impedance increases is a fourth trend. In an example, the fourth trend represents that the second invalidity data increases slowly or remains unchanged as the contact impedance increases, indicating that when the contact impedance is smaller than the first impedance threshold $K_1'$, the contact impedance has a relatively small impact on the EEG signal. In this case, the degree of validity of the EEG signal is relatively high.

When the contact impedance is greater than or equal to the first impedance threshold $K_1'$ and smaller than a second impedance threshold $K_2'$, a trend in which the second invalidity data increases as the contact impedance increases is a fifth trend. In an example, the fifth trend represents that the second invalidity data increases rapidly as the contact impedance increases, indicating that when the contact impedance is greater than or equal to the first impedance threshold $K_1'$ and smaller than the second impedance threshold $K_2'$, the contact impedance has a relatively large impact on the EEG signal. In this case, the degree of validity of the EEG signal is relatively low.

When the contact impedance is greater than or equal to the second impedance threshold $K_2'$ and smaller than a third impedance threshold $K_3'$, a trend in which the second invalidity data increases as the contact impedance increases is a sixth trend. In an example, the sixth trend represents that the second invalidity data increases more rapidly as the contact impedance increases, indicating that when the contact impedance is greater than or equal to the second impedance threshold $K_2'$ and smaller than the third impedance threshold $K_3'$, the contact impedance has a huge impact on the EEG signal. In this case, the degree of validity of the EEG signal is very low.

The first impedance threshold $K_1'$ is smaller than the second impedance threshold $K_2'$, and the second impedance threshold $K_2'$ is smaller than the third impedance threshold $K_3'$. The fourth trend is smaller than the fifth trend, and the fifth trend is smaller than the sixth trend.

FIG. 8 is a graph of a second predetermined relationship according to an embodiment of the present disclosure.

As shown in FIG. 8, the second predetermined relationship may include, for example, a functional relationship between the contact impedance (such as an impedance value) and the second invalidity data $V_2$, which is, for example, a piecewise function. FIG. 8 shows that the piecewise function has three intervals.

In a first interval of the function, the contact impedance is smaller than the first impedance threshold $K_1'$, and the second invalidity data $V_2$ remains unchanged as the contact impedance increases, indicating that the contact impedance has a small impact on the EEG signal. In this case, the degree of validity of the EEG signal is relatively high.

In a second interval of the function, the contact impedance is greater than or equal to the first impedance threshold $K_1'$ and smaller than the second impedance threshold $K_2'$, and the second invalidity data $V_2$ increases rapidly as the contact impedance increases, indicating that the contact impedance has a large impact on the EEG signal. In this case, the degree of validity of the EEG signal is relatively low.

In a third interval of the function, the contact impedance is greater than or equal to the second impedance threshold $K_2'$ and smaller than the third impedance threshold $K_3'$, and the second invalidity data $V_2$ increases more rapidly as the contact impedance increases, indicating that the contact impedance has a huge impact on the EEG signal. In this case, the degree of validity of the EEG signal is very low.

It can be understood that in the embodiments of the present disclosure, the second invalidity data is determined based on the different signal intensity thresholds and distinguished based on the different situations. In this way, accuracy of the second invalidity data is improved. Therefore, the verification effect of EEG signal is improved.

After the first invalidity data and the second invalidity data are obtained, a first weight of the first invalidity data may be determined, and a second weight of the second invalidity data may be determined. Then, first data is obtained by multiplying the first invalidity data by the first weight, second data is obtained by multiplying the second invalidity data by the second weight, and the first data and the second data are added to obtain the comprehensive invalidity data

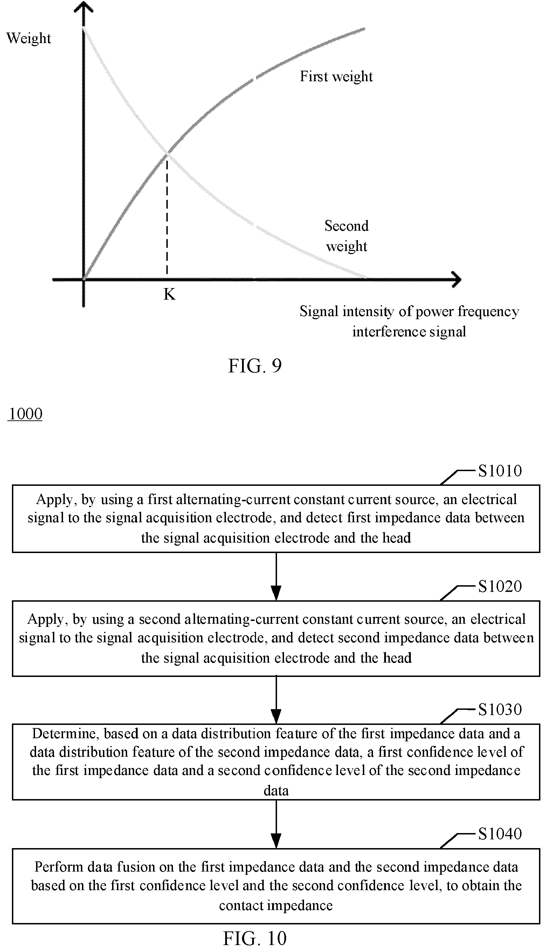

FIG. 9 is a graph of a first weight and a second weight according to an embodiment of the present disclosure.

As shown in FIG. 9, when the signal intensity of the power frequency interference signal is greater than predetermined threshold value K, the first weight is greater than the second weight. When the signal intensity of the power frequency interference signal is smaller than or equal to the predetermined threshold value K, the first weight is smaller than or equal to the second weight. A sum of the first weight and the second weight is 1.

In an embodiment, the first invalidity data and the second invalidity data are fused based on the first weight and the second weight, to obtain the comprehensive invalidity data, for example, as shown in formula (8):

$$V = P_1 * V_1 + P_2 * V_2 \qquad (8)$$

where $V_1$ represents the first invalidity data; $V_2$ represents the second invalidity data; $P_1$ represents the first weight, $P_2$ represents the second weight; and V represents the comprehensive invalidity data.

It can be understood that when the signal intensity of the power frequency interference signal is relatively large, the power frequency interference signal has a relatively large impact on the EEG signal. Therefore, the signal intensity of the power frequency interference signal is mainly considered during weight allocation. In practice, since the impact of the power frequency interference signal on the EEG signal is typically greater than the impact of the contact impedance on the EEG signal, the weight allocation is required when the first invalidity data and the second invalidity data are fused. During the weight allocation, when the signal intensity of the power frequency interference signal is relatively high, the validity of the EEG signal is mainly determined by the signal intensity of the power frequency interference signal; and when the signal intensity of the power frequency interference signal is relatively low, the validity of the EEG signal is mainly determined by the impedance. It can be seen that the weight allocation based on the signal intensity of the power frequency interference signal improves a fusion effect. In this way, the comprehensive invalidity data obtained by the fusion can more accurately reflect the validity of the EEG signal.

The present disclosure further provides a human intelligence-based impedance detection method.

FIG. 10 is a schematic flowchart of a human intelligence-based impedance detection method according to an embodiment of the present disclosure.

As shown in FIG. 10, a human intelligence-based impedance detection method 1000 according to embodiments of the present disclosure includes actions at steps S1010 to S1040.

At step S1010, an electrical signal is applied to the signal acquisition electrode by using a first alternating-current constant current source, and first impedance data between the signal acquisition electrode and the head is detected.

At step S1020, an electrical signal is applied to the signal acquisition electrode by using a second alternating-current constant current source, and second impedance data between the signal acquisition electrode and the head is detected.

At step S1030, a first confidence level of the first impedance data and a second confidence level of the second impedance data are determined based on a data distribution feature of the first impedance data and a data distribution feature of the second impedance data.

At step S1040, data fusion is performed on the first impedance data and the second impedance data based on the first confidence level and the second confidence level, to obtain the contact impedance.

In an embodiment, the first impedance data between the signal acquisition electrode and the head includes first impedance data between the signal acquisition electrode and a scalp. The first alternating-current constant current source differs from the second alternating-current constant current source. Both the first alternating-current constant current source and the second alternating-current constant current source may be used to apply a constant current electrical signal. In an embodiment, a frequency of the electrical signal applied by the first alternating-current constant current source differs from a frequency of the electrical signal applied by the second alternating-current constant current source. For example, a frequency of the electrical signal applied by the first alternating-current constant current source is greater than a frequency of the electrical signal applied by the second alternating-current constant current source. For example, the frequency of the electrical signal applied by the first alternating-current constant current source may be about 80 Hz, and the frequency of the electrical signal applied by the second alternating-current constant current source may be about 10 Hz, about 20 Hz, about 30 Hz, about 40 Hz, etc.

In an embodiment, the first alternating-current constant current source is provided by an integrated chip for measuring the EEG signal. That is, the first alternating-current constant current source is directly provided by the integrated chip for measuring the EEG signal rather than being provided additionally. In this way, cost of impedance detection apparatus is reduced.

In an embodiment, the second alternating-current constant current source may be provided by a Wien bridge self-excited oscillation circuit. For example, the Wien bridge self-excited oscillation circuit may generate a sinusoidal wave signal that may be applied to the signal acquisition electrode.

In an embodiment, the first alternating-current constant current source applies an electrical signal to the signal acquisition electrode and a reference electrode, and the second alternating-current constant current source applies an electrical signal to the signal acquisition electrode and the reference electrode. The reference electrode is generally configured to determine the signal reference.

After the first impedance data and the second impedance data are detected, the first confidence level of the first impedance data and the second confidence level of the second impedance data are determined based on a data distribution feature of the first impedance data and a data distribution feature of the second impedance data. Concentrated impedance data distribution with lower dispersion degree correspond to a higher confidence level.

After the first confidence and the second confidence are obtained, the first impedance data and the second impedance data may be fused based on the first confidence and the second confidence to obtain the contact impedance. Compared with first impedance data and second impedance data obtained by a single alternating-current constant current source, the contact impedance more accurately reflects the impedance between the signal acquisition electrode and the head. It can be seen that the impedance detection method of the present disclosure can improve accuracy of the impedance detection.

As shown in FIG. 5 above, the first alternating-current constant current source is provided by an integrated chip. In FIG. 5, DVSS represents Digital VSS, which is ground of a digital part of a circuit; AVDD represents an analog voltage, which powers an analog device in the chip; each of LOFF_SENSP, LOFF_SENSN, and FLEAD_OFF represents a register; ADC represents an analog-to-digital converter; BIAS OUT pin is provided with a bias current. A chip protection circuit prevents an excessive current through a resistor.

$Z_1$, $Z_2$, and $Z_3$ represent impedances between the signal acquisition electrode and the head (the scalp). Resistance exists when the signal acquisition electrode is in contact with the scalp, and therefore $Z_1$, $Z_2$, and $Z_3$ represent impedances.

As shown in FIG. 6, the second alternating-current constant current source may be provided by a Wien bridge self-excited oscillation circuit. FIG. 6 shows an example of the Wien bridge self-excited oscillation circuit for providing a sinusoidal wave signal and applying the sinusoidal wave signal to the signal acquisition electrode and the reference electrode.

In an embodiment, impedance data is detected at a plurality of first time points, and impedance data detected at adjacent time points are selected from the impedance data detected at the plurality of first time points as the first impedance data. For example, 100 pieces of impedance data are detected at 100 time points, and 10 pieces of impedance data corresponding to last 10 time points are selected as the first impedance data. The time points corresponding to the last 10 pieces of impedance data are most recent time points. Since the impedance changes over time, the 10 pieces of impedance data at the adjacent time points has better timeliness. Similarly, impedance data is detected at a plurality of second time points, and impedance data detected at adjacent time points are selected from the impedance data detected at the plurality of second time point as the second impedance data. For example, the plurality of second time points also include 100 time points, and impedance data corresponding to 10 adjacent time points are selected as the second impedance data. Both the first impedance data and the second impedance data are normally distributed.

It can be understood that the accuracy of the impedance detection is improved by selecting the impedance data with the better timeliness for subsequent processing.

In an embodiment, the data distribution feature includes a data discretization feature. A first deviation value of the first impedance data may be determined based on a data discretization feature of the first impedance data; a second deviation value of the second impedance data may be determined based on a data discretization feature of the second impedance data; and the first confidence level and the second confidence level may be determined based on the first deviation value and the second deviation value.

For example, the first confidence level is determined based on the first deviation value and the second deviation value; and the second confidence level is determined based on the first confidence level and a predetermined constraint condition. The predetermined constraint condition includes a sum of the first confidence level and the second confidence level being 1.

In an embodiment, as shown in formula (9), the first impedance data is represented as $x_1$, a first deviation value of first impedance data $x_1$ conforming to a normal distribution includes a first variance $\sigma_1^2$, $\mu_1$ is a mean of the first impedance data $x_1$, and the first impedance data $x_1$ is estimated by maximum likelihood to obtain the mean $\mu_1$ and the first variance $\sigma_1^2$.

$$\mu_1 = \overline{x_1}, \quad \sigma_1^2 = \frac{1}{n}\sum_{i=1}^{n}\left(x_{1_i} - \mu_1\right)^2 \quad (9)$$

where n represents the number of data in the first impedance data $x_1$. As described above, when the first impedance data $x_1$ includes 10 pieces of impedance data, n=10.

As shown in formula (10), the second impedance data is represented as $x_2$, a second deviation value of the second impedance data $x_2$ conforming to a normal distribution includes a second variance $\sigma_2^2$, $\mu_2$ is a mean of the second impedance data $x_2$, and the second impedance data $x_2$ is estimated by maximum likelihood to obtain the mean $\mu_2$ and the first variance $\sigma_2^2$.

$$\mu_2 = \overline{x_2}, \quad \sigma_2^2 = \frac{1}{n}\sum_{i=1}^{n}\left(x_{2_i} - \mu_2\right)^2 \quad (10)$$

where n represents the number of data in the second impedance data $x_2$. As mentioned above, when the second impedance data $x_2$ includes 10 pieces of impedance data, n=10.

As shown in formula (11), $\alpha$ represents a first confidence level; $\beta$ represents a second confidence level; and a sum of the first confidence level $\alpha$ and the second confidence level $\beta$ is 1. Fused impedance data $\hat{x}$ represents a fusion relationship between the first impedance data $x_1$ and the second impedance data $x_2$. The fused impedance data $\hat{x}$ is obtained by fusing the first impedance data $x_1$ and the second impedance data $x_2$.

$$\hat{x} = \alpha x_1 + \beta x_2$$
$$\alpha + \beta = 1 \qquad (11)$$
$$\Rightarrow \hat{x} = \alpha x_1 + (1-\alpha) x_2$$

After the first variance $\sigma_1^2$ and the second variance $\sigma_2^2$ are obtained, a functional relationship between fused variance $\hat{\sigma}^2$ and the first confidence level a may be determined based on the first variance $\sigma_1^2$ and the second variance $\sigma_2^2$, which is shown in formula (12). The fused variance $\hat{\sigma}^2$ is a variance of the fused impedance data $\hat{x}$.

$$\hat{\sigma}^2(\alpha) = (1-\alpha)^2 \sigma_1^2 + \alpha^2 \sigma_2^2 \quad (12)$$

A derivative of the first confidence level $\alpha$ is taken based on the functional relationship between the fused variance $\hat{\sigma}^2$ and the first confidence level a, and the first confidence level a is obtained when the fused variance $\hat{\sigma}^2$ is minimized, as shown in formula (13).

$$\alpha = \frac{\sigma_1^2}{\sigma_1^2 + \sigma_2^2} \quad (13)$$

The second confidence level $\beta$ is obtained based on a constraint relationship between the first confidence level $\alpha$ and the second confidence level $\beta$, as shown in formula (14).

$$\beta = \frac{\sigma_2^2}{\sigma_1^2 + \sigma_2^2} \quad (14)$$

As shown in formula (15), the first data is obtained by multiplying the first confidence level a by the first impedance data $x_1$, the second data is obtained by multiplying the second confidence level $\beta$ by the second impedance data $x_2$, and the first data and the second data are added to obtain the fused impedance data $\hat{x}$. When the fused impedance data $\hat{x}$ conforms to the normal distribution, a mean of the fused impedance data $x_2$ is determined as a final contact impedance.

$$\hat{x}(x_1, x_2) = \frac{\sigma_1^2}{\sigma_1^2 + \sigma_2^2} x_1 + \frac{\sigma_2^2}{\sigma_1^2 + \sigma_2^2} x_2 \quad (15)$$

The present disclosure also provides a human intelligence-based EEG signal verification method.

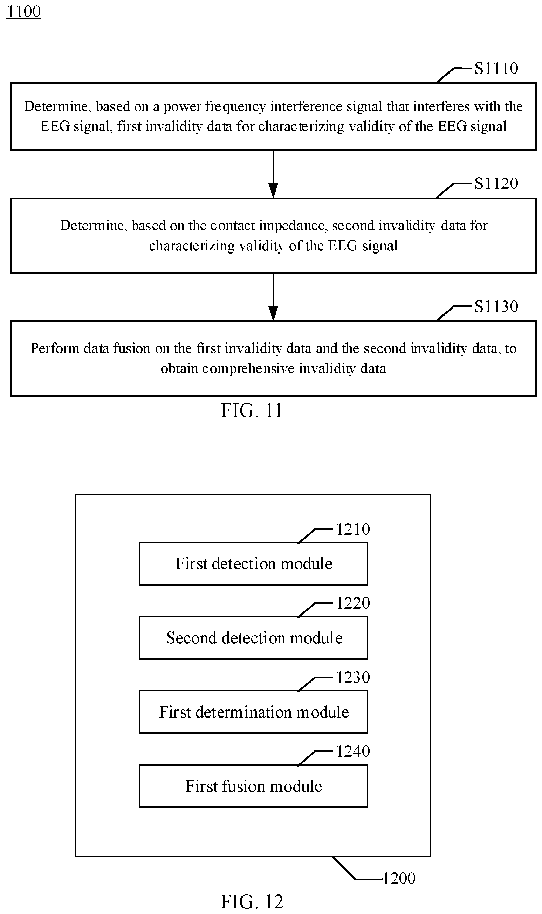

FIG. 11 is a schematic flowchart of a human intelligence-based EEG signal verification method according to an embodiment of the present disclosure.

As shown in FIG. 11, a human intelligence-based EEG signal verification method 1100 according to an embodiment of the present disclosure includes, for example, actions at steps S1110 to S1130.

At step S1110, first invalidity data for characterizing validity of the EEG signal is determined based on a power frequency interference signal that interferes with the EEG signal.

In an embodiment, the power frequency interference signal includes, for example, an environmental noise signal. In the process of acquiring the EEG signal, the environmental noise may interfere with the acquisition process, resulting in fusion of the power frequency interference signal into the acquired EEG signal, which reduces validity of the EEG signal. Therefore, the power frequency interference signal may be separated from the acquired EEG signal, and first invalidity data characterizing the validity of the EEG signal may be determined based on the power frequency interference signal.

At step S1120, second invalidity data for characterizing validity of the EEG signal is determined based on the contact impedance.

In an embodiment, the contact impedance includes an impedance between the signal acquisition electrode and the head, and the signal acquisition electrode is configured to acquire the EEG signal. The impedance between the signal acquisition electrode and the head includes, for example, the impedance between the signal acquisition electrode and the head scalp. When the EEG signal is acquired, the signal acquisition electrode is attached to the scalp. In this case, an impedance is formed between the signal acquisition electrode and the scalp. The contact impedance may be, for example, an impedance value. A greater impedance indicates a worse contact between the signal acquisition electrode and the scalp, and lower quality of the acquired EEG signal. Therefore, the second invalidity data characterizing the validity of the EEG signal may be determined based on the contact impedance.

At step S1130, data fusion is performed on the first invalidity data and the second invalidity data, to obtain comprehensive invalidity data.

In an embodiment, the first invalidity data and the second invalidity data that characterize the validity of the EEG signal are obtained in different manners, and obtained fused invalidity data can more comprehensively and accurately represent the validity of the EEG signal. It can be seen that the validity of the EEG signal is comprehensively verified based on the power frequency interference signal and the contact impedance, to achieve comprehensive verification from multiple perspective. In this way, verification accuracy and verification effect are improved.

In an embodiment, the first invalidity data may be determined based on a signal intensity of the power frequency interference signal and a first predetermined relationship. The first predetermined relationship characterizes a relationship between the signal intensity of the power frequency interference signal and the first invalidity data.

In an embodiment, the EEG signal may be filtered based on a predetermined frequency to obtain a filtered EEG signal. A frequency of the environmental noise interference signal is typically around 50 Hz. Therefore, the predetermined frequency used in the filtering includes 50 Hz, or may be around 50 Hz, such as 40 Hz to 60 Hz. A recursive filter (IIR digital filter) may be used for the filtering.

After the filtering, the power frequency interference signal is obtained based on a difference between the EEG signal before the filtering and the filtered EEG signal; and a root mean square of the power frequency interference signal is calculated as the signal intensity of the power frequency interference signal. Greater signal intensity of the power frequency interference signal indicates a higher degree of the power frequency interference during the EEG signal acquisition. In this case, the first invalidity data indicates a higher degree of invalidity of the EEG signal.

In an embodiment, when the signal intensity of the power frequency interference signal is smaller than a first signal intensity threshold $K_1$, a trend in which the first invalidity data increases as the signal intensity of the power frequency interference signal increases is a first trend. In an example, the first trend represents that the first invalidity data increases slowly or remains unchanged as the signal intensity of the power frequency interference signal increases, indicating that when the signal intensity of the power frequency interference signal is smaller than first signal intensity threshold $K_1$, the power frequency interference signal has a relatively small impact on the EEG signal. In this case, a degree of validity of the EEG signal is relatively high.

When the signal intensity of the power frequency interference signal is greater than or equal to the first signal intensity threshold $K_1$ and smaller than a second signal intensity threshold $K_2$, a trend in which the first invalidity data increases as the signal intensity of the power frequency interference signal increases is a second trend. In an example, the second trend represents that the first invalidity data increases rapidly as the signal intensity of the power frequency interference signal increases, indicating that when the signal intensity of the power frequency interference signal is greater than or equal to the first signal intensity threshold $K_1$ and smaller than the second signal intensity threshold $K_2$, the power frequency interference signal has a relatively large impact on the EEG signal. In this case, the degree of validity of the EEG signal is relatively low.

When the signal intensity of the power frequency interference signal is greater than or equal to the second signal intensity threshold $K_2$ and smaller than a third signal intensity threshold $K_3$, a trend in which the first invalidity data increases as the signal intensity of the power frequency interference signal increases is a third trend. In an example, the third trend indicates that the first invalidity data increases more rapidly as the signal intensity of the power frequency interference signal increases, indicating that when the signal intensity of the power frequency interference signal is greater than or equal to second signal intensity threshold $K_2$ and smaller than the third signal intensity threshold $K_3$, the power frequency interference signal has a huge impact on the EEG signal. In this case, the degree of validity of the EEG signal is very low.

The first signal intensity threshold $K_1$ is smaller than the second signal intensity threshold $K_2$, and the second signal intensity threshold $K_2$ is smaller than the third signal intensity threshold $K_3$. The first trend is smaller than the second trend, and the second trend is smaller than the third trend.

As shown in FIG. 7 above, the first predetermined relationship may include, for example, a functional relationship between the signal intensity of the power frequency interference signal and the first invalidity data $V_1$, which is, for example, a piecewise function. FIG. 7 shows that the piecewise function has three intervals.

In a first interval of the function, the signal intensity of the power frequency interference signal is smaller than the first signal intensity threshold $K_1$, and the first invalidity data $V_1$ remains unchanged as the signal intensity of the power frequency interference signal increases, indicating that the power frequency interference signal has a relatively small impact on the EEG signal. In this case, the degree of validity of the EEG signal is relatively high.

In a second interval of the function, the signal intensity of the power frequency interference signal is greater than or equal to the first signal intensity threshold $K_1$ and smaller than the second signal intensity threshold $K_2$, and the first invalidity data $V_1$ increases rapidly as the signal intensity of the power frequency interference signal increases, indicating that the power frequency interference signal has a relatively large impact on the EEG signal. In this case, the degree of validity of the EEG signal is relatively low.

In a third interval of the function, the signal intensity of the power frequency interference signal is greater than or equal to the second signal intensity threshold $K_2$ and smaller than the third signal intensity threshold $K_3$, and the first invalidity data $V_1$ increases more rapidly as the signal intensity of the power frequency interference signal increases, indicating that the power frequency interference signal has a huge impact on the EEG signal. In this case, the degree of validity of the EEG signal is very low.

It can be understood that in the embodiments of the present disclosure, the first invalidity data is determined based on the different signal intensity thresholds and distinguished based on the different situations. In this way, accuracy of the first invalidity data is improved. Therefore, the verification effect of EEG signal is improved.

In an embodiment, the second invalidity data is determined based on the contact impedance and a second predetermined relationship. The second predetermined relationship characterizes a relationship between the contact impedance and the second invalidity data.

In an embodiment, when the contact impedance is smaller than a first impedance threshold $K_1$', a trend in which the second invalidity data increases as the contact impedance increases is a fourth trend. In an example, the fourth trend represents that the second invalidity data increases slowly or remains unchanged as the contact impedance increases, indicating that when the contact impedance is smaller than the first impedance threshold $K_1$', the contact impedance has a relatively small impact on the EEG signal. In this case, the degree of validity of the EEG signal is relatively high.

When the contact impedance is greater than or equal to the first impedance threshold $K_1$' and smaller than a second impedance threshold $K_2$', a trend in which the second invalidity data increases as the contact impedance increases is a fifth trend. In an example, the fifth trend represents that the second invalidity data increases rapidly as the contact impedance increases, indicating that when the contact impedance is greater than or equal to the first impedance threshold $K_1$' and smaller than the second impedance threshold $K_2$', the contact impedance has a relatively large impact on the EEG signal. In this case, the degree of validity of the EEG signal is relatively low.

When the contact impedance is greater than or equal to the second impedance threshold $K_2$' and smaller than a third impedance threshold $K_3$', a trend in which the second invalidity data increases as the contact impedance increases is a sixth trend. In an example, the sixth trend represents that the second invalidity data increases more rapidly as the contact impedance increases, indicating that when the contact impedance is greater than or equal to the second impedance threshold $K_2$' and smaller than the third impedance threshold $K_3$', the contact impedance has a huge impact on the EEG signal. In this case, the degree of validity of the EEG signal is very low.

The first impedance threshold $K_1$' is smaller than the second impedance threshold $K_2$', and the second impedance threshold $K_2$' is smaller than the third impedance threshold $K_3$'. The fourth trend is smaller than the fifth trend, and the fifth trend is smaller than the sixth trend.

As shown in FIG. 8 above, the second predetermined relationship may include, for example, a functional relationship between the contact impedance (such as an impedance value) and the second invalidity data $V_2$, which is, for example, a piecewise function. FIG. 8 shows that the piecewise function has three intervals.

In a first interval of the function, the contact impedance is smaller than the first impedance threshold $K_1'$, and the second invalidity data $V_2$ remains unchanged as the contact impedance increases, indicating that the contact impedance has a small impact on the EEG signal. In this case, the degree of validity of the EEG signal is relatively high.

In a second interval of the function, the contact impedance is greater than or equal to the first impedance threshold $K_1'$ and smaller than the second impedance threshold $K_2'$, and the second invalidity data $V_2$ increases rapidly as the contact impedance increases, indicating that the contact impedance has a large impact on the EEG signal. In this case, the degree of validity of the EEG signal is relatively low.

In a third interval of the function, the contact impedance is greater than or equal to the second impedance threshold $K_2'$ and smaller than the third impedance threshold $K_3'$, and the second invalidity data $V_2$ increases more rapidly as the contact impedance increases, indicating that the contact impedance has a huge impact on the EEG signal. In this case, the degree of validity of the EEG signal is very low.

It can be understood that in the embodiments of the present disclosure, the second invalidity data is determined based on the different signal intensity thresholds and distinguished based on the different situations. In this way, accuracy of the second invalidity data is improved. Therefore, the verification effect of EEG signal is improved.

After the first invalidity data and the second invalidity data are obtained, a first weight of the first invalidity data may be determined, and a second weight of the second invalidity data may be determined. Then, the first data is obtained by multiplying the first invalidity data by the first weight, the second data is obtained by multiplying the second invalidity data by the second weight, and the first data and the second data are added to obtain the comprehensive invalidity data

As shown in FIG. 9 above, when the signal intensity of the power frequency interference signal is greater than predetermined threshold value K, the first weight is greater than the second weight. When the signal intensity of the power frequency interference signal is smaller than or equal to the predetermined threshold value K, the first weight is smaller than or equal to the second weight. A sum of the first weight and the second weight is 1.

In an embodiment, the first invalidity data and the second invalidity data are fused based on the first weight and the second weight, to obtain the comprehensive invalidity data, for example, as shown in formula (16):

$$V = P_1 * V_1 + P_2 * V_2 \qquad (16)$$

where $V_1$ represents the first invalidity data; $V_2$ represents the second invalidity data; $P_1$ represents the first weight, $P_2$ represents the second weight; and V represents the comprehensive invalidity data.

It can be understood that when the signal intensity of the power frequency interference signal is relatively large, the power frequency interference signal has a relatively large impact on the EEG signal. Therefore, the signal intensity of the power frequency interference signal is mainly considered during weight allocation. In practice, since the impact of the power frequency interference signal on the EEG signal is typically greater than the impact of the contact impedance on the EEG signal, the weight allocation is required when the first invalidity data and the second invalidity data are fused. During the weight allocation, when the signal intensity of the power frequency interference signal is relatively high, the validity of the EEG signal is mainly determined by the signal intensity of the power frequency interference signal; and when the signal intensity of the power frequency interference signal is relatively low, the validity of the EEG signal is mainly determined by the impedance. It can be seen that the weight allocation based on the signal intensity of the power frequency interference signal improves a fusion effect. In this way, the comprehensive invalidity data obtained by the fusion can more accurately reflect the validity of the EEG signal.

FIG. 12 is a schematic diagram of a human intelligence-based impedance detection apparatus according to an embodiment of the present disclosure.

As shown in FIG. 12, a human intelligence-based impedance detection apparatus 1200 according to an embodiment of the present disclosure includes a first detection module 1210, a second detection module 1220, a first determination module 1230, and a first fusion module 1240.

The first detection module 1210 is configured to apply, by using a first alternating-current constant current source, an electrical signal to a signal acquisition electrode, and detect first impedance data between the signal acquisition electrode and the head.

The second detection module 1220 is configured to apply, by using a second alternating-current constant current source, an electrical signal to the signal acquisition electrode, and detect second impedance data between the signal acquisition electrode and the head.

The first determination module 1230 is configured to determine, based on a data distribution feature of the first impedance data and a data distribution feature of the second impedance data, a first confidence level for the first impedance data and a second confidence level for the second impedance data.

The first fusion module 1240 is configured to perform data fusion on the first impedance data and the second impedance data based on the first confidence level and the second confidence level, to obtain a contact impedance.

In an embodiment, a frequency of the electrical signal applied by the first alternating-current constant current source differs from a frequency of the electrical signal applied by the second alternating-current constant current source. The first alternating-current constant current source is provided by an integrated chip configured to measure the EEG signal, and the second alternating-current constant current source is provided by a Wien bridge self-excited oscillation circuit configured to generate a sinusoidal wave.

In an embodiment, impedance data is detected at a plurality of first time points, and impedance data detected at adjacent time points are selected from the impedance data detected at the plurality of first time points as the first impedance data; and impedance data is detected at a plurality of second time points, and impedance data detected at adjacent time points are selected from the impedance data detected at the plurality of second time point as the second impedance data. The first impedance data is normally distributed, and the second impedance data is normally distributed.

In an embodiment, the data distribution feature includes data discretization feature; and the first determination module 1230 is further configured to: determine a first deviation value of the first impedance data based on a data discretization feature of the first impedance data; determine a second deviation value of the second impedance data based on a data discretization feature of the second impedance data; and determine the first confidence level and the second confidence level based on the first deviation value and the second deviation value.

In an embodiment, the determining the first confidence level and the second confidence level based on the first deviation value and the second deviation value includes: determining the first confidence level based on the first deviation value and the second deviation value; and determining the second confidence level based on the first confidence level and a predetermined constraint condition.

In an embodiment, the first deviation value includes a first variance of first impedance data conforming to a normal distribution, and the second deviation value includes a second variance of second impedance data conforming to a normal distribution; and the determining the first confidence level based on the first deviation value and the second deviation value includes: determining a functional relationship between a fused variance and the first confidence level based on the first variance and the second variance, where the fusion variance is a variance of fused impedance data, the fused impedance data representing a fusion relationship between the first impedance data and the second impedance data; and taking a derivative of the first confidence level based on the functional relationship between the fused variance and the first confidence level, and obtaining the first confidence level when the fusion variance is minimized.

In an embodiment, the predetermined constraint condition includes a sum of the first confidence level and the second confidence level being 1.

In an embodiment, the first fusion module 1240 is further configured to: obtain first data by multiplying the first confidence level by the first impedance data, obtain second data by multiplying the second confidence level by the second impedance data, and add the first data and the second data to obtain fused impedance data; determine, when the fused impedance data conforms to a normal distribution, a mean of the fused impedance data as the contact impedance.

In an embodiment, the first alternating-current constant current source applies the electrical signal to the signal acquisition electrode and a reference electrode, and the second alternating-current constant current source applies the electrical signal to the signal acquisition electrode and the reference electrode.

It can be understood that, for the specific implementation process of the human intelligence-based impedance detection apparatus 1200, reference can be made to the implementation process of the human intelligence-based impedance detection method above, and details thereof will be omitted herein.

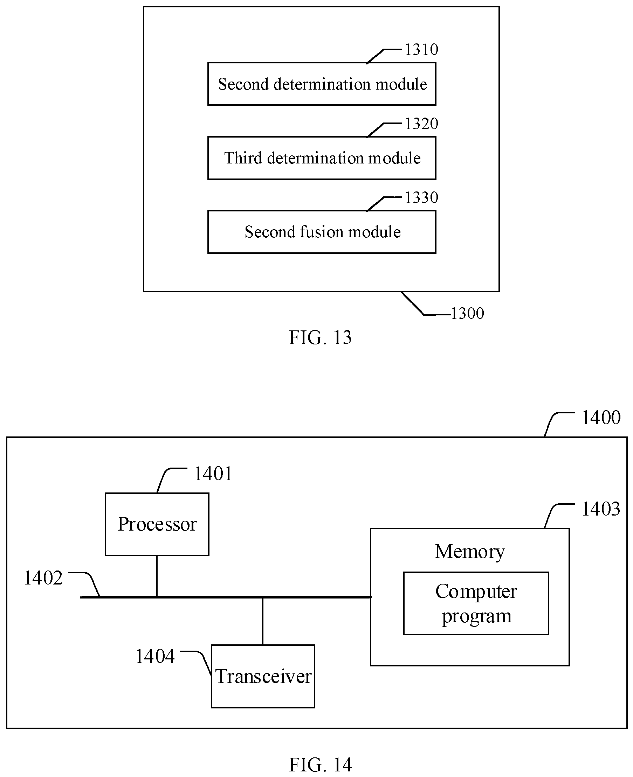

FIG. 13 is a schematic diagram of a human intelligence-based EEG signal verification apparatus according to an embodiment of the present disclosure.

As shown in FIG. 13, a human intelligence-based EEG signal verification apparatus 1300 according to an embodiment of the present disclosure includes a second determination module 1310, a third determination module 1320, and a second fusion module 1330.

The second determination module 1310 is configured to determine, based on a power frequency interference signal that interferes with an EEG signal, first invalidity data for characterizing validity of the EEG signal.

The third determination module 1320 is configured to determine, based on a contact impedance, second invalidity data for characterizing validity of the EEG signal. The contact impedance includes an impedance between a signal acquisition electrode and a head, and the signal acquisition electrode is configured to acquire the EEG signal.

The second fusion module 1330 is configured to perform data fusion on the first invalidity data and the second invalidity data, to obtain comprehensive invalidity data.

In an embodiment, the second determination module 1310 is further configured to determine the first invalidity data based on a signal intensity of the power frequency interference signal and a first predetermined relationship. The first predetermined relationship characterizes a relationship between the signal intensity of the power frequency interference signal and the first invalidity data.

In an embodiment, the signal intensity of the power frequency interference signal is obtained by: filtering the EEG signal based on a predetermined frequency to obtain a filtered EEG signal; obtaining the power frequency interference signal based on a difference between the EEG signal before the filtering and the filtered EEG signal; and calculating a root mean square of the power frequency interference signal as the signal intensity of the power frequency interference signal.

In an embodiment, the predetermined frequency includes 50 Hz.

In an embodiment, the first predetermined relationship includes: when the signal intensity of the power frequency interference signal is smaller than a first signal intensity threshold, a trend in which the first invalidity data increases as the signal intensity of the power frequency interference signal increases being a first trend; when the signal intensity of the power frequency interference signal is greater than or equal to the first signal intensity threshold and smaller than a second signal intensity threshold, a trend in which the first invalidity data increases as the signal intensity of the power frequency interference signal increases being a second trend; and when the signal intensity of the power frequency interference signal is greater than or equal to the second signal intensity threshold and smaller than a third signal intensity threshold, a trend in which the first invalidity data increases as the signal intensity of the power frequency interference signal increases being a third trend. The first signal intensity threshold is smaller than the second signal intensity threshold, and the second signal intensity threshold is smaller than the third signal intensity threshold. The first trend is smaller than the second trend, and the second trend is smaller than the third trend.

In an embodiment, the third determination module 1320 is further configured to determine the second invalidity data based on the contact impedance and a second predetermined relationship. The second predetermined relationship characterizes a relationship between the contact impedance and the second invalidity data.

In an embodiment, the second predetermined relationship includes: when the contact impedance is smaller than a first impedance threshold, a trend in which the second invalidity data increases as the contact impedance increases being a fourth trend; when the contact impedance is greater than or equal to the first impedance threshold and smaller than a second impedance threshold, a trend in which the second invalidity data increases as the contact impedance increases being a fifth trend; when the contact impedance is greater than or equal to the second impedance threshold and smaller than a third impedance threshold, a trend in which the second invalidity data increases as the contact impedance increases being a sixth trend. The first impedance threshold is smaller than the second impedance threshold, and the second impedance threshold is smaller than the third impedance threshold. The fourth trend is smaller than the fifth trend, and the fifth trend is smaller than the sixth trend.

In an embodiment, the second fusion module 1330 is configured to determine a first weight of the first invalidity data; determine a second weight of the second invalidity data; obtain first data by multiplying the first invalidity data by the first weight, obtain second data by multiplying the second invalidity data by the second weight, and add the first data and the second data to obtain the comprehensive invalidity data.

In an embodiment, when the signal intensity of the power frequency interference signal is greater than a predetermined threshold, the first weight is greater than the second weight; when the signal intensity of the power frequency interference signal is smaller than or equal to the predetermined threshold, the first weight is smaller than or equal to the second weight; and a sum of the first weight and the second weight is 1.

It can be understood that, for the specific implementation process of the human intelligence-based EEG signal verification apparatus 1300, reference can be made to the implementation process of the human intelligence-based EEG signal verification method above, and details thereof will be omitted herein.

Embodiments of the present disclosure provide a computer-readable storage medium. The medium has a computer program stored thereon. The computer program, when executed by a processor, implements the steps of each of the methods in any of the above embodiments.

An implementation of the present disclosure provides a computer program product. The computer program product includes instructions. The instructions are configured to be executed by a processor of a computer device to cause the computer device to perform the steps of each of the methods in any of the above embodiments.

FIG. 14 is a structural block diagram of an electronic device according to an embodiment of the present disclosure. As shown in FIG. 14, an electronic device 1400 includes a processor 1401 and a memory 1403. The processor 1401 is connected to the memory 1403, for example, via a bus 1402. In another exemplary embodiment of the present disclosure, the electronic device 1400 may further include a transceiver 1404. It should be noted that in practical application, the electronic device 1400 is not limited to include one transceiver 1404, and a structure of the electronic device 1400 does not constitute a limitation on the embodiments of the present disclosure.

The processor 1401 may be a Central Processing Unit (CPU), a general-purpose processor, a Digital Signal Processor (DSP), an Application Specific Integrated Circuit (ASIC), a Field-Programmable Gate Array (FPGA) or other programmable logic devices, transistor logic devices, hardware components, or any combination thereof, which can realize or execute various exemplary logic blocks, modules, and circuits described in the present disclosure. The processor 1401 can also be a combination that achieves a computing function, such as a combination of one or more microprocessors, a combination of a DSP and a microprocessor, etc.

The bus 1402 can include a pathway for transmitting information between the above components. The bus 1402 can be a PCI (Peripheral Component Interconnect) bus, or an EISA (Extended Industry Standard Architecture) bus, etc. The bus 1402 can be divided into an address bus, a data bus, a control bus, etc. For ease of representation, only one thick line is used in FIG. 14, but it does not mean that there is only one bus or one type of bus.

Memory 1403 stores a computer program corresponding to each of the methods according to the above embodiments of the present disclosure. The computer program is controlled and executed by the processor 1401. The processor 1401 is configured to execute a computer program stored in the memory 1403 to implement the method embodiments shown above.

The electronic device 1400 includes, but is not limited to, mobile terminals such as mobile phones, laptop computers, digital broadcast receivers, PDAs (personal digital assistants), PADs (tablet computers), and PMPs (portable multimedia players), and fixed terminals such as digital TVs and desktop computers. The electronic device 1400 shown in FIG. 14 is merely an example and should not impose any limitation to the functions and scope of use of the embodiments of the present disclosure.

It should be noted that the logic and/or step described in other manners herein or shown in the flow chart, for example, a particular sequence list of executable instructions for realizing the logical function, may be specifically achieved in any computer readable medium to be used by the instruction execution system, device or equipment (such as the system based on computers, the system comprising processors or other systems capable of obtaining the instruction from the instruction execution system, device and equipment and executing the instruction), or to be used in combination with the instruction execution system, device and equipment. As to the specification, "the computer readable medium" may be any device adaptive for including, storing, communicating, propagating or transferring programs to be used by or in combination with the instruction execution system, device or equipment. More specific examples of the computer readable medium comprise but are not limited to: an electronic connection (an electronic device) with one or more wires, a portable computer enclosure (a magnetic device), a random access memory (RAM), a read only memory (ROM), an erasable programmable read-only memory (EPROM or a flash memory), an optical fiber device and a portable compact disk read-only memory (CDROM). In addition, the computer readable medium may even be a paper or other appropriate medium capable of printing programs thereon, this is because, for example, the paper or other appropriate medium may be optically scanned and then edited, decrypted or processed with other appropriate methods when necessary to obtain the programs in an electric manner, and then the programs may be stored in the computer memories.

It should be understood that each part of the present disclosure may be realized by the hardware, software, firmware or their combination. In the above embodiments, a plurality of steps or methods may be realized by the software or firmware stored in the memory and executed by the appropriate instruction execution system. For example, if it is realized by the hardware, likewise in another embodiment, the steps or methods may be realized by one or a combination of the following techniques known in the art: a discrete logic circuit having a logic gate circuit for realizing a logic function of a data signal, an application-specific integrated circuit having an appropriate combination logic gate circuit, a programmable gate array (PGA), a field programmable gate array (FPGA), etc.

Reference throughout this specification to "an embodiment", "some embodiments", "an example", "a specific example", or "some examples" means that a particular feature, structure, material, or characteristic described in connection with the embodiment or example is included in at least one embodiment or example of the present disclosure. The schematic representations of the above terms do not necessarily refer to the same embodiment or example. Moreover, the described specific features, structures, materials or characteristics may be combined in any one or more embodiments or examples in a suitable manner.

In addition, the terms such as "first" and "second" are used herein for purposes of description and are not intended to indicate or imply relative importance, or to implicitly show the number of technical features indicated. Thus, the feature defined with "first" and "second" may explicitly or implicitly comprise one or more this feature. In the description of the present disclosure, "a plurality of" means at least two, for example, two or three, unless specified otherwise.

In the present disclosure, unless specified or limited otherwise, the terms "mounted," "connected," "coupled" and "fixed" are understood broadly, such as fixed, detachable mountings, connections and couplings or integrated, and may be mechanical or electrical mountings, connections and couplings, and also may be direct and via media indirect mountings, connections, and couplings, and further may be inner mountings, connections and couplings of two components or interaction relations between two components. For those skilled in the art, the specific meaning of the above-mentioned terms in the embodiments of the present disclosure can be understood according to specific circumstances.

Although embodiments of present disclosure have been shown and described above, it should be understood that above embodiments are just explanatory, and cannot be construed to limit the present disclosure, for those skilled in the art, changes, alternatives, and modifications can be made to the embodiments without departing from spirit, principles and scope of the present disclosure.

**Claims**

1. A computer-implemented method for controlling a signal acquisition electrode (03), wherein the signal acquisition electrode (03) is movably connected to a wearable component (01) of an electroencephalogram, EEG, signal acquisition apparatus, the wearable component (01) being adapted to be worn on a head of a user, and the EEG signal acquisition apparatus further comprising a pushing component (02); and wherein the method comprises:

    detecting (S401) a contact impedance between the signal acquisition electrode (03) and the head, wherein said detecting (S401) the contact impedance comprises:

        applying (S1010), by using a first alternating-current constant current source, an electrical signal to the signal acquisition electrode (03), and detecting first impedance data between the signal acquisition electrode (03) and the head;
        applying (S1020), by using a second alternating-current constant current source, an electrical signal to the signal acquisition electrode (03), and detecting second impedance data between the signal acquisition electrode (03) and the head;
        determining (S1030), based on a data distribution feature of the first impedance data and a data distribution feature of the second impedance data, a first confidence level for the first impedance data and a second confidence level for the second impedance data; and
        performing (S1040) data fusion on the first impedance data and the second impedance data based on the first confidence level and the second confidence level, to obtain the contact impedance; and

    controlling (S403), when the contact impedance between the signal acquisition electrode (03) and the head is greater than or equal to a first impedance threshold, the pushing component (02) to push the signal acquisition electrode (03) to move towards the head to acquire an EEG signal by using the signal acquisition electrode (03).

2. The method according to claim 1, wherein said controlling (S403) the pushing component (02) to push the signal acquisition electrode (03) to move towards the head comprises:

    determining, from a correspondence between impedances and intensities, a reference pushing intensity corresponding to the contact impedance; and controlling the pushing component (02) to push, in accordance with the reference pushing intensity, the signal acquisition electrode (03) to move towards the head, wherein a plurality of impedances are recorded in the correspondence, and any two of the plurality of impedances differ from each other; or
    determining a target pushing intensity of the pushing component (02) based on a difference between the first impedance threshold and the contact impedance; and controlling the pushing component (02) to push, in accordance with the target pushing intensity, the signal acquisition electrode (03) to move towards the head.

3. The method according to claim 2, wherein said determining the target pushing intensity of the pushing component (02) based on the difference between the first impedance threshold and the contact impedance comprises:
processing the difference between the first impedance threshold and the contact impedance with a proportional-integral-derivative algorithm, to obtain the target pushing intensity of the pushing component (02).

4. The method according to any one of claims 1 to 3, wherein the EEG signal acquisition apparatus comprises a plurality of signal acquisition electrodes (03), and the method further comprises:
acquiring, by using the plurality of signal acquisition electrodes (03), the EEG signal in response to a contact impedance of each of a target number of signal acquisition electrodes (03) among the plurality of signal acquisition electrodes (03) satisfying a target condition, wherein:

    the target number is smaller than or equal to a total number of the plurality of signal acquisition electrodes (03) and is greater than a number threshold; and
    the target condition comprises a duration during which the contact impedance is smaller than the first impedance threshold reaching a target duration.

5. The method according to claim 1, wherein:

    a frequency of the electrical signal applied by the first alternating-current constant current source differs from a frequency of the electrical signal applied by the second alternating-current constant current source, the first alternating-current constant current source is provided by an integrated chip configured to measure the EEG signal, and the second alternating-current constant current source being provided by a Wien bridge self-excited oscillation circuit configured to generate a sinusoidal wave; and/or

the first alternating-current constant current source applies the electrical signal to the signal acquisition electrode (03) and a reference electrode, and the second alternating-current constant current source applies the electrical signal to the signal acquisition electrode (03) and the reference electrode; and/or

impedance data is detected at a plurality of first time points, and impedance data detected at adjacent time points are selected from the impedance data detected at the plurality of first time points as the first impedance data; and impedance data is detected at a plurality of second time points, and impedance data detected at adjacent time points are selected from the impedance data detected at the plurality of second time point as the second impedance data, the first impedance data being normally distributed, and the second impedance data being normally distributed.

6. The method according to claim 1, wherein the data distribution feature comprises data discretization feature, and said determining (S 1030), based on the data distribution feature of the first impedance data and the data distribution feature of the second impedance data, the first confidence level of the first impedance data and the second confidence level of the second impedance data comprises:

   determining a first deviation value of the first impedance data based on a data discretization feature of the first impedance data;
   determining a second deviation value of the second impedance data based on a data discretization feature of the second impedance data; and
   determining the first confidence level and the second confidence level based on the first deviation value and the second deviation value.

7. The method according to claim 6, wherein said determining the first confidence level and the second confidence level based on the first deviation value and the second deviation value comprises:

   determining the first confidence level based on the first deviation value and the second deviation value; and

determining the second confidence level based on the first confidence level and a predetermined constraint condition, the predetermined constraint condition comprising a sum of the first confidence level and the second confidence level being 1.

8. The method according to claim 7, wherein the first deviation value comprises a first variance of first impedance data conforming to a normal distribution, and the second deviation value comprises a second variance of second impedance data conforming to a normal distribution; and

said determining the first confidence level based on the first deviation value and the second deviation value comprises:

   determining a functional relationship between a fused variance and the first confidence level based on the first variance and the second variance, wherein the fusion variance is a variance of fused impedance data, the fused impedance data representing a fusion relationship between the first impedance data and the second impedance data; and
   taking a derivative of the first confidence level based on the functional relationship between the fused variance and the first confidence level, and obtaining the first confidence level when the fusion variance is minimized.

9. The method according to claim 1, wherein said performing (S1040) the data fusion on the first impedance data and the second impedance data based on the first confidence level and the second confidence level, to obtain the contact impedance comprises:

   obtaining first data by multiplying the first confidence level by the first impedance data, obtaining second data by multiplying the second confidence level by the second impedance data, and adding the first data and the second data to obtain fused impedance data;
   determining, when the fused impedance data conforms to a normal distribution, a mean of the fused impedance data as the contact impedance.

10. The method according to any one of claims 1 to 9, further comprising:
    verifying the EEG signal subsequent to the EEG signal having been acquired by the signal acquisition electrode (03), wherein said verifying the EEG signal comprises:

    determining (S1110), based on a power frequency interference signal that interferes with the EEG signal, first invalidity data for characterizing validity of the EEG signal;

determining (S1120), based on the contact impedance, second invalidity data for characterizing validity of the EEG signal; and

performing (S1130) data fusion on the first invalidity data and the second invalidity data, to obtain comprehensive invalidity data.

11. The method according to claim 10, wherein said determining (S1110), based on the power frequency interference signal that interferes with the EEG signal, the first invalidity data for characterizing the validity of the EEG signal comprises:

determining the first invalidity data based on a signal intensity of the power frequency interference signal and a first predetermined relationship, the first predetermined relationship characterizing a relationship between the signal intensity of the power frequency interference signal and the first invalidity data;

or wherein said determining, based on the contact impedance, the second invalidity data for characterizing the validity of the EEG signal comprises:

determining the second invalidity data based on the contact impedance and a second predetermined relationship, the second predetermined relationship characterizing a relationship between the contact impedance and the second invalidity data.

12. The method according to claim 11, wherein the signal intensity of the power frequency interference signal is obtained by: filtering the EEG signal based on a predetermined frequency to obtain a filtered EEG signal, the predetermined frequency comprising 50 Hz; obtaining the power frequency interference signal based on a difference between the EEG signal before the filtering and the filtered EEG signal; and calculating a root mean square of the power frequency interference signal as the signal intensity of the power frequency interference signal; and/or the first predetermined relationship comprises:

when the signal intensity of the power frequency interference signal is smaller than a first signal intensity threshold, a trend in which the first invalidity data increases as the signal intensity of the power frequency interference signal increases being a first trend;

when the signal intensity of the power frequency interference signal is greater than or equal to the first signal intensity threshold and smaller than a second signal intensity threshold, a trend in which the first invalidity data increases as the signal intensity of the power frequency interference signal increases being a second trend; and when the signal intensity of the power frequency interference signal is greater than or equal to the second signal intensity threshold and smaller than a third signal intensity threshold, a trend in which the first invalidity data increases as the signal intensity of the power frequency interference signal increases being a third trend, wherein: the first signal intensity threshold is smaller than the second signal intensity threshold, and the second signal intensity threshold is smaller than the third signal intensity threshold; and the first trend is smaller than the second trend, and the second trend is smaller than the third trend;

or wherein the second predetermined relationship comprises:

when the contact impedance is smaller than a first impedance threshold, a trend in which the second invalidity data increases as the contact impedance increases being a fourth trend;

when the contact impedance is greater than or equal to the first impedance threshold and smaller than a second impedance threshold, a trend in which the second invalidity data increases as the contact impedance increases being a fifth trend;

when the contact impedance is greater than or equal to the second impedance threshold and smaller than a third impedance threshold, a trend in which the second invalidity data increases as the contact impedance increases being a sixth trend, wherein: the first impedance threshold is smaller than the second impedance threshold, and the second impedance threshold is smaller than the third impedance threshold; and the fourth trend is smaller than the fifth trend, and the fifth trend is smaller than the sixth trend.

13. The method according to any one of claims 10 to 12, wherein said performing (S1130) the data fusion on the first invalidity data and the second invalidity data to obtain the comprehensive invalidity data comprises:

determining a first weight of the first invalidity data;

determining a second weight of the second invalidity data;

obtaining first data by multiplying the first invalidity data by the first weight, obtaining second data by multiplying the second invalidity data by the second weight, and adding the first data and the second data to obtain the comprehensive invalidity data, wherein:

when the signal intensity of the power frequency interference signal is greater than a predetermined threshold, the first weight is greater than the second weight;

when the signal intensity of the power frequency interference signal is smaller than or equal to the predetermined threshold, the first weight is smaller than or equal to the second weight; and

a sum of the first weight and the second weight is 1.

14. An electronic device (1400) for controlling a signal acquisition electrode (03), wherein the signal acquisition electrode (03) is movably connected to a wearable component (01) of an electroencephalogram, EEG, signal acquisition apparatus, the wearable component (01) being adapted to be worn on a head of a user, and the EEG signal acquisition apparatus further comprising a pushing component (02), and wherein the electronic device (1400) comprises:

a memory (1403);
a processor (1401); and
a computer program stored in the memory (1403) and executable by the processor (1401), wherein the processor (1401), when executing the computer program, implements the method according to any one of claims 1 to 13.

**Patentansprüche**

1. Computerimplementiertes Verfahren zum Steuern einer Signalerfassungselektrode (03), wobei die Signalerfassungselektrode (03) beweglich mit einer tragbaren Komponente (01) einer Vorrichtung zur Erfassung von Elektroenzephalogramm-(EEG-)Signalen verbunden ist, wobei die tragbare Komponente (01) dazu ausgebildet ist, auf dem Kopf eines Benutzers getragen zu werden, und wobei die Vorrichtung zur Erfassung von EEG-Signalen ferner eine Druckkomponente (02) umfasst; und wobei das Verfahren Folgendes umfasst:
Erfassen (S401) einer Kontaktimpedanz zwischen der Signalerfassungselektrode (03) und dem Kopf, wobei das Erfassen (S401) der Kontaktimpedanz umfasst:

Anlegen (S1010) eines elektrischen Signals an die Signalerfassungselektrode (03) unter Verwendung einer ersten Wechselstrom-Konstantstromquelle und Erfassen erster Impedanzdaten zwischen der Signalerfassungselektrode (03) und dem Kopf;
Anlegen (S1020) eines elektrischen Signals an die Signalerfassungselektrode (03) unter Verwendung einer zweiten Wechselstrom-Konstantstromquelle und Erfassen zweiter Impedanzdaten zwischen der Signalerfassungselektrode (03) und dem Kopf;
Bestimmen (S1030) eines ersten Konfidenzniveaus für die ersten Impedanzdaten und eines zweiten Konfidenzniveaus für die zweiten Impedanzdaten auf der Grundlage eines Datenverteilungsmerkmals der ersten Impedanzdaten und eines Datenverteilungsmerkmals der zweiten Impedanzdaten; und
Durchführen (S1040) einer Datenfusion der ersten Impedanzdaten und der zweiten Impedanzdaten auf der Grundlage des ersten Konfidenzniveaus und des zweiten Konfidenzniveaus, um die Kontaktimpedanz zu erhalten; und
Steuern (S403) der Druckkomponente (02), wenn die Kontaktimpedanz zwischen der Signalerfassungselektrode (03) und dem Kopf größer oder gleich einer ersten Impedanzschwelle ist, um die Signalerfassungselektrode (03) in Richtung auf den Kopf zu drücken, sodass unter Verwendung der Signalerfassungselektrode (03) ein EEG-Signal erfasst wird.

2. Verfahren nach Anspruch 1, wobei das Steuern (S403) der Druckkomponente (02), um die Signalerfassungselektrode (03) in Richtung auf den Kopf zu drücken, umfasst:

Bestimmen aus einer Zuordnung zwischen Impedanzen und Intensitäten, einer der Kontaktimpedanz entsprechenden Referenzdruckintensität; und Steuern der Druckkomponente (02), um die Signalerfassungselektrode (03) gemäß der Referenzdruckintensität in Richtung auf den Kopf zu drücken, wobei in der Zuordnung mehrere Impedanzen gespeichert sind und sich beliebige zwei der mehreren Impedanzen voneinander unterscheiden; oder
Bestimmen einer Zieldruckintensität der Druckkomponente (02) basierend auf einer Differenz zwischen der ersten Impedanzschwelle und der Kontaktimpedanz; und Steuern der Druckkomponente (02), um die Signalerfassungselektrode (03) gemäß der Zieldruckintensität in Richtung auf den Kopf zu drücken.

3. Verfahren nach Anspruch 2, wobei das Bestimmen der Zieldruckintensität der Druckkomponente (02) basierend auf der Differenz zwischen der ersten Impedanzschwelle und der Kontaktimpedanz umfasst:
Verarbeiten der Differenz zwischen der ersten Impedanzschwelle und der Kontaktimpedanz mit einem Proportional-Integral-Derivative-Algorithmus, um die Zieldruckintensität der Druckkomponente (02) zu erhalten.

4. Verfahren nach einem der Ansprüche 1 bis 3, wobei die Vorrichtung zur Erfassung von EEG-Signalen mehrere Signalerfassungselektroden (03) umfasst und das Verfahren ferner umfasst:

Erfassen des EEG-Signals unter Verwendung der mehreren Signalerfassungselektroden (03) als Reaktion darauf, dass eine Kontaktimpedanz jeder Signalerfassungselektrode (03) einer Zielanzahl von Signalerfassungselektroden (03) unter den mehreren Signalerfassungselektroden (03) eine Zielbedingung erfüllt, wobei:

die Zielanzahl kleiner als oder gleich der Gesamtzahl der mehreren Signalerfassungselektroden (03) und größer als eine Anzahlschwelle ist; und

Die Zielbedingung eine Dauer umfasst, in der die Kontaktimpedanz kleiner als die erste Impedanzschwelle ist, die eine Zieldauer erreicht.

5. Verfahren nach Anspruch 1, wobei eine Frequenz des von der ersten Wechselstrom-Konstantstromquelle angelegten elektrischen Signals sich von einer Frequenz des von der zweiten Wechselstrom-Konstantstromquelle angelegten elektrischen Signals unterscheidet, wobei die erste Wechselstrom-Konstantstromquelle von einem integrierten Chip bereitgestellt wird, der dazu konfiguriert ist, das EEG-Signal zu messen, und wobei die zweite Wechselstrom-Konstantstromquelle von einer Wien-Brücken-Selbsterregungsschaltung bereitgestellt wird, die dazu konfiguriert ist, eine Sinuswelle zu erzeugen; und/oder

wobei die erste Wechselstrom-Konstantstromquelle das elektrische Signal an die Signalerfassungselektrode (03) und eine Referenzelektrode anlegt und die zweite Wechselstrom-Konstantstromquelle das elektrische Signal an die Signalerfassungselektrode (03) und die Referenzelektrode anlegt; und/oder
wobei Impedanzdaten an einer Vielzahl von ersten Zeitpunkten erfasst werden und die an benachbarten Zeitpunkten erfassten Impedanzdaten als erste Impedanzdaten aus den an der Vielzahl von ersten Zeitpunkten erfassten Impedanzdaten ausgewählt werden; und wobei Impedanzdaten an einer Vielzahl von zweiten Zeitpunkten erfasst werden und die an benachbarten Zeitpunkten erfasste Impedanzdaten als zweite Impedanzdaten aus den an der Vielzahl von zweiten Zeitpunkten erfassten Impedanzdaten ausgewählt werden; wobei die ersten Impedanzdaten normalverteilt sind, und die zweiten Impedanzdaten normalverteilt sind.

6. Verfahren nach Anspruch 1, wobei das Datenverteilungsmerkmal ein Datendiskretisierungsmerkmal umfasst, und wobei das Bestimmen (S1030) eines ersten Konfidenzniveaus für die ersten Impedanzdaten und eines zweiten Konfidenzniveaus für die zweiten Impedanzdaten auf der Grundlage des Datenverteilungsmerkmals der ersten Impedanzdaten und des Datenverteilungsmerkmals der zweiten Impedanzdaten umfasst:

Bestimmen eines ersten Abweichungswerts der ersten Impedanzdaten basierend auf einem Datendiskretisierungsmerkmal der ersten Impedanzdaten;
Bestimmen eines zweiten Abweichungswerts der zweiten Impedanzdaten basierend auf einem Datendiskretisierungsmerkmal der zweiten Impedanzdaten; und
Bestimmen des ersten und zweiten Konfidenzniveaus basierend auf dem ersten und zweiten Abweichungswert.

7. Verfahren gemäß Anspruch 6, wobei das Bestimmen des ersten Konfidenzniveaus und des zweiten Konfidenzniveaus basierend auf dem ersten Abweichungswert und dem zweiten Abweichungswert umfasst:

Bestimmen des ersten Konfidenzniveaus basierend auf dem ersten und zweiten Abweichungswert; und
Bestimmen des zweiten Konfidenzniveaus basierend auf dem ersten Konfidenzniveau und einer vorbestimmten Randbedingung, wobei die vorbestimmte Randbedingung umfasst, dass eine Summe des ersten Konfidenzniveaus und des zweiten Konfidenzniveaus 1 ist.

8. Verfahren nach Anspruch 7, wobei der erste Abweichungswert eine erste Varianz der ersten Impedanzdaten, die einer Normalverteilung entsprechen, umfasst, und der zweite Abweichungswert eine zweite Varianz der zweiten Impedanzdaten, die einer Normalverteilung entsprechen, umfasst; und
das Bestimmen des ersten Konfidenzniveaus basierend auf dem ersten und zweiten Abweichungswert umfasst:

Bestimmen einer funktionellen Beziehung zwischen einer Fusionsvarianz und dem ersten Konfidenzniveaus basierend auf der ersten Varianz und der zweiten Varianz, wobei die Fusionsvarianz eine Varianz von fusionierten Impedanzdaten ist, die fusionierten Impedanzdaten eine Fusionsbeziehung zwischen den ersten Impedanzdaten und den zweiten Impedanzdaten repräsentieren; und
Bilden einer Ableitung des ersten Konfidenzniveaus basierend auf der funktionalen Beziehung zwischen der Fusionsvarianz und dem ersten Konfidenzniveau und Ermitteln des ersten Konfidenzniveaus, wenn die Fusionsvarianz minimiert ist.

9. Verfahren nach Anspruch 1, wobei das Durchführen (S1040) der Datenfusion der ersten Impedanzdaten und der zweiten Impedanzdaten auf der Grundlage des ersten Konfidenzniveaus und des zweiten Konfidenzniveaus, um die Kontaktimpedanz zu erhalten, umfasst:

Ermitteln erster Daten durch Multiplizieren des ersten Konfidenzniveaus mit den ersten Impedanzdaten, Ermitteln zweiter Daten durch Multiplizieren des zweiten Konfidenzniveaus mit den zweiten Impedanzdaten und Addieren der ersten Daten und der zweiten Daten, um fusionierte Impedanzdaten zu erhalten;
Bestimmen, wenn die fusionierten Impedanzdaten einer Normalverteilung entsprechen, eines Mittelwerts der fusionierten Impedanzdaten als die Kontaktimpedanz.

10. Verfahren nach einem der Ansprüche 1 bis 9, das ferner umfasst:
Verifizieren des EEG-Signals nachdem das EEG-Signal durch die Signalerfassungselektrode (03) erfasst wurde, wobei das Verifizieren des EEG-Signals umfasst:

Bestimmen (S1110) erster Ungültigkeitsdaten zur Charakterisierung der Gültigkeit des EEG-Signals auf der Grundlage eines Netzfrequenzstörsignals, das das EEG-Signal stört;
Bestimmen (S1120) zweiter Ungültigkeitsdaten zur Charakterisierung der Gültigkeit des EEG-Signals auf der Grundlage der Kontaktimpedanz; und
Durchführen (S1130) einer Datenfusion der ersten Ungültigkeitsdaten und der zweiten Ungültigkeitsdaten, um umfassende Ungültigkeitsdaten zu erhalten.

11. Verfahren nach Anspruch 10, wobei das Bestimmen (S1110) der ersten Ungültigkeitsdaten zur Charakterisierung der Gültigkeit des EEG-Signals auf der Grundlage des Netzfrequenzstörsignals, das das EEG-Signal stört, umfasst:

Bestimmen der ersten Ungültigkeitsdaten basierend auf einer Signalintensität des Netzfrequenzstörsignals und einer ersten vorbestimmten Beziehung, wobei die erste vorbestimmte Beziehung eine Beziehung zwischen der Signalintensität des Netzfrequenzstörsignals und den ersten Ungültigkeitsdaten charakterisiert.
oder wobei das Bestimmen der zweiten Ungültigkeitsdaten zur Charakterisierung der Gültigkeit des EEG-Signals auf der Grundlage der Kontaktimpedanz umfasst:
Bestimmen der zweiten Ungültigkeitsdaten basierend auf der Kontaktimpedanz und einer zweiten vorbestimmten Beziehung, wobei die zweite vorbestimmte Beziehung eine Beziehung zwischen der Kontaktimpedanz und den zweiten Ungültigkeitsdaten charakterisiert.

12. Verfahren nach Anspruch 11, wobei die Signalintensität des Netzfrequenzstörsignals durch Folgendes ermittelt wird: Filtern des EEG-Signals basierend auf einer vorbestimmten Frequenz, um ein gefiltertes EEG-Signal zu erhalten, wobei die vorbestimmte Frequenz 50 Hz umfasst; Ermitteln des Netzfrequenzstörsignals basierend auf einer Differenz zwischen dem EEG-Signal vor dem Filtern und dem gefilterten EEG-Signal; und Berechnen eines quadratischen Mittelwerts des Netzfrequenzstörsignals als Signalintensität des Netzfrequenzstörsignals; und/oder wobei die erste vorbestimmte Beziehung Folgendes umfasst:

wenn die Signalintensität des Netzfrequenzstörsignals kleiner als eine erste Signalintensitätsschwelle ist, ist der Trend, bei dem die ersten Ungültigkeitsdaten mit zunehmender Signalintensität des Netzfrequenzstörsignals ansteigen, ein erster Trend;
wenn die Signalintensität des Netzfrequenzstörsignals größer oder gleich der ersten Signalintensitätsschwelle und kleiner als eine zweite Signalintensitätsschwelle ist, ist der Trend, bei dem die ersten Ungültigkeitsdaten mit zunehmender Signalintensität des Netzfrequenzstörsignals ansteigen, ein zweiter Trend;
wenn die Signalintensität des Netzfrequenzstörsignals größer oder gleich der zweiten Signalintensitätsschwelle und kleiner als eine dritte Signalintensitätsschwelle ist, ist der Trend, bei dem die ersten Ungültigkeitsdaten mit zunehmender Signalintensität des Netzfrequenzstörsignals ansteigen, ein dritter Trend; wobei: die erste Signalintensitätsschwelle kleiner ist als die zweite Signalintensitätsschwelle und die zweite Signalintensitätsschwelle kleiner ist als die dritte Signalintensitätsschwelle; und der erste Trend kleiner ist als der zweite Trend und der zweite Trend kleiner ist als der dritte Trend;
oder wobei die zweite vorbestimmte Beziehung umfasst:
wenn die Kontaktimpedanz kleiner als eine erste Impedanzschwelle ist, ist der Trend, bei dem die zweiten Ungültigkeitsdaten mit zunehmender Kontaktimpedanz ansteigen, ein vierter Trend; wenn die Kontaktimpedanz größer als oder gleich der ersten Impedanzschwelle und kleiner als eine zweite Impedanzschwelle ist, ist der Trend, bei dem die zweiten Ungültigkeitsdaten mit zunehmender Kontaktimpedanz ansteigen, ein fünfter Trend;
wenn die Kontaktimpedanz größer als oder gleich der zweiten Impedanzschwelle und kleiner als eine dritte Impedanzschwelle ist, ist der Trend, bei dem die zweiten Ungültigkeitsdaten mit zunehmender Kontaktimpedanz ansteigen, ein sechster Trend; wobei: die erste Impedanzschwelle kleiner ist als die zweite Impedanzschwelle und die zweite Impedanzschwelle kleiner ist als die dritte Impedanzschwelle; und der vierte Trend kleiner ist als der fünfte Trend und der fünfte Trend kleiner ist als der sechste Trend.

13. Verfahren nach einem der Ansprüche 10 bis 12, wobei das Durchführen (S1130) der Datenfusion der ersten Ungültigkeitsdaten und der zweiten Ungültigkeitsdaten, um die umfassenden Ungültigkeitsdaten zu erhalten, Folgendes umfasst:

Bestimmen einer ersten Gewichtung der ersten Ungültigkeitsdaten;
Bestimmen einer zweiten Gewichtung der zweiten Ungültigkeitsdaten;
Ermitteln erster Daten durch Multiplizieren der ersten Ungültigkeitsdaten mit der ersten Gewichtung, Ermitteln zweiter Daten durch Multiplizieren der zweiten Ungültigkeitsdaten mit der zweiten Gewichtung und Addieren der ersten Daten und der zweiten Daten, um die umfassenden Ungültigkeitsdaten zu erhalten, wobei:

wenn die Signalintensität des Netzfrequenzstörsignals größer ist als eine vorbestimmte Schwelle, ist die erste Gewichtung größer als die zweite Gewichtung;
wenn die Signalintensität des Netzfrequenzstörsignals kleiner oder gleich der vorbestimmten Schwelle ist, ist die erste Gewichtung kleiner oder gleich der zweiten Gewichtung; und
wobei eine Summe der ersten Gewichtung und der zweiten Gewichtung 1 ist.

14. Elektronisches Gerät (1400) zum Steuern einer Signalerfassungselektrode (03), wobei die Signalerfassungselektrode (03) beweglich mit einer tragbaren Komponente (01) einer Vorrichtung zur Erfassung von EEG-Signalen verbunden ist, wobei die tragbare Komponente (01) dazu ausgelegt ist, am Kopf eines Benutzers getragen zu werden, und die Vorrichtung zur Erfassung von EEG-Signalen ferner eine Druckkomponente (02) umfasst, und wobei das elektronische Gerät (1400) umfasst:

einen Speicher (1403);
einen Prozessor (1401); und
ein in dem Speicher (1403) gespeichertes und durch den Prozessor (1401) ausführbares Computerprogramm,
wobei der Prozessor (1401) bei Ausführen des Computerprogramms das Verfahren nach einem der Ansprüche 1 bis 13 implementiert.

## Revendications

1. Un procédé mis en œuvre par ordinateur pour commander une électrode d'acquisition de signal (03), dans lequel l'électrode d'acquisition de signal (03) est connectée de manière mobile à un composant portable (01) d'un appareil d'acquisition de signal d'électroencéphalogramme (EEG), le composant portable (01) étant adapté pour être porté sur la tête d'un utilisateur, et l'appareil d'acquisition de signal EEG comprenant en outre un composant de poussée (02) ; et dans lequel le procédé comprend :
la détection (S401) d'une impédance de contact entre l'électrode d'acquisition de signal (03) et la tête, dans lequel ladite détection (S401) de l'impédance de contact comprend :

l'application (S1010), au moyen d'une première source de courant constant alternatif, d'un signal électrique à l'électrode d'acquisition de signal (03), et la détection de premières données d'impédance entre l'électrode d'acquisition de signal (03) et la tête ;
l'application (S1020), au moyen d'une seconde source de courant constant alternatif, d'un signal électrique à l'électrode d'acquisition de signal (03), et la détection de secondes données d'impédance entre l'électrode d'acquisition de signal (03) et la tête ;
la détermination (S1030), basée sur une caractéristique de distribution de données des premières données d'impédance et d'une caractéristique de distribution de données des secondes données d'impédance, d'un premier niveau de confiance pour les premières données d'impédance et d'un second niveau de confiance pour les secondes données d'impédance ; et
la réalisation (S1040) d'une fusion de données sur les premières données d'impédance et les secondes données d'impédance, basée sur le premier niveau de confiance et le second niveau de confiance, pour obtenir l'impédance de contact ; et
la commande (S403), lorsque l'impédance de contact entre l'électrode d'acquisition de signal (03) et la tête est supérieure ou égale à un premier seuil d'impédance, du composant de poussée (02) pour pousser l'électrode d'acquisition de signal (03) à se déplacer vers la tête, afin d'acquérir un signal EEG au moyen de l'électrode d'acquisition de signal (03).

2. Le procédé selon la revendication 1, dans lequel ladite commande (S403) du composant de poussée (02) pour pousser l'électrode d'acquisition de signal (03) à se déplacer vers la tête comprend :

la détermination, à partir d'une correspondance entre des impédances et des intensités, d'une intensité de poussée de référence correspondant à l'impédance de contact ; et la commande du composant de poussée (02) pour pousser, conformément à l'intensité de poussée de référence, l'électrode d'acquisition de signal (03) à se déplacer vers la tête, dans lequel une pluralité d'impédances sont enregistrées dans la correspondance, et deux impédances quelconques de la pluralité diffèrent l'une de l'autre ; ou la détermination d'une intensité de poussée cible du composant de poussée (02), basée sur une différence entre le premier seuil d'impédance et l'impédance de contact ; et la commande du composant de poussée (02) pour pousser, conformément à l'intensité de poussée cible, l'électrode d'acquisition de signal (03) à se déplacer vers la tête.

3.  Le procédé selon la revendication 2, dans lequel ladite détermination de l'intensité de poussée cible du composant de poussée (02), basée sur la différence entre le premier seuil d'impédance et l'impédance de contact, comprend :
    le traitement de la différence entre le premier seuil d'impédance et l'impédance de contact à l'aide d'un algorithme proportionnel-intégral-dérivé, afin d'obtenir l'intensité de poussée cible du composant de poussée (02).

4.  Le procédé selon l'une quelconque des revendications 1 à 3, dans lequel l'appareil d'acquisition de signal EEG comprend une pluralité d'électrodes d'acquisition de signal (03), et le procédé comprend en outre :
    l'acquisition, au moyen de la pluralité d'électrodes d'acquisition de signal (03), du signal EEG, lorsque l'impédance de contact de chacune d'un nombre cible d'électrodes d'acquisition de signal (03) parmi la pluralité d'électrodes d'acquisition de signal (03) satisfait une condition cible, dans lequel :

    le nombre cible est inférieur ou égal au nombre total de la pluralité d'électrodes d'acquisition de signal (03) et est supérieur à un seuil numérique ; et
    la condition cible comprend qu'une durée pendant laquelle l'impédance de contact est inférieure au premier seuil d'impédance atteint une durée cible.

5.  Le procédé selon la revendication 1, dans lequel :

une fréquence du signal électrique appliqué par la première source de courant constant alternatif diffère d'une fréquence du signal électrique appliqué par la seconde source de courant constant alternatif, la première source de courant constant alternatif étant fournie par une puce intégrée configurée pour mesurer le signal EEG, et la seconde source de courant constant alternatif étant fournie par un circuit d'oscillation auto-excitée à pont de Wien configuré pour générer une onde sinusoïdale ; et/ou
la première source de courant constant alternatif applique le signal électrique à l'électrode d'acquisition de signal (03) et à une électrode de référence, et la seconde source de courant constant alternatif applique le signal électrique à l'électrode d'acquisition de signal (03) et à l'électrode de référence ; et/ou
des données d'impédance sont détectées à une pluralité de premiers instants, et des données d'impédance détectées à des instants adjacents sont sélectionnées parmi les données d'impédance détectées à la pluralité de premiers instants en tant que premières données d'impédance ; et des données d'impédance sont détectées à une pluralité de seconds instants, et des données d'impédance détectées à des instants adjacents sont sélectionnées parmi les données d'impédance détectées à la pluralité de seconds instants en tant que secondes données d'impédance, les premières données d'impédance étant distribuées normalement, et les secondes données d'impédance étant distribuées normalement.

6.  Le procédé selon la revendication 1, dans lequel la caractéristique de distribution de données comprend une caractéristique de discrétisation de données, et ladite détermination (S1030), basée sur la caractéristique de distribution de données des premières données d'impédance et de la caractéristique de distribution de données des secondes données d'impédance, du premier niveau de confiance des premières données d'impédance et du second niveau de confiance des secondes données d'impédance comprend :

la détermination d'une première valeur d'écart des premières données d'impédance, basée sur une caractéristique de discrétisation de données des premières données d'impédance ;
la détermination d'une seconde valeur d'écart des secondes données d'impédance, basée sur une caractéristique de discrétisation de données des secondes données d'impédance ; et
la détermination du premier niveau de confiance et du second niveau de confiance, basée sur la première valeur d'écart et de la seconde valeur d'écart.

**7.** Le procédé selon la revendication 6, dans lequel ladite détermination du premier niveau de confiance et du second niveau de confiance, basée sur la première valeur d'écart et de la seconde valeur d'écart, comprend :

la détermination du premier niveau de confiance, basée sur la première valeur d'écart et de la seconde valeur d'écart ; et
la détermination du second niveau de confiance, basée sur le premier niveau de confiance et d'une condition de contrainte prédéterminée, la condition de contrainte prédéterminée comprenant que la somme du premier niveau de confiance et du second niveau de confiance est égale à 1.

**8.** Le procédé selon la revendication 7, dans lequel la première valeur d'écart comprend une première variance de premières données d'impédance conformes à une distribution normale, et la seconde valeur d'écart comprend une deuxième variance de secondes données d'impédance conformes à une distribution normale ; et

ladite détermination du premier niveau de confiance, basée sur la première valeur d'écart et de la seconde valeur d'écart, comprend :

la détermination d'une relation fonctionnelle entre une variance fusionnée et le premier niveau de confiance, basée sur la première variance et la deuxième variance, dans lequel la variance fusionnée est une variance de données d'impédance fusionnées, les données d'impédance fusionnées représentant une relation de fusion entre les premières données d'impédance et les secondes données d'impédance ; et
le calcul d'une dérivée du premier niveau de confiance, basée sur la relation fonctionnelle entre la variance fusionnée et le premier niveau de confiance, et l'obtention du premier niveau de confiance lorsque la variance fusionnée est minimisée.

**9.** Le procédé selon la revendication 1, dans lequel ladite réalisation (S1040) de la fusion de données sur les premières données d'impédance et les secondes données d'impédance, basée sur le premier niveau de confiance et le second niveau de confiance, pour obtenir l'impédance de contact, comprend :

l'obtention de premières données en multipliant le premier niveau de confiance par les premières données d'impédance, l'obtention de secondes données en multipliant le second niveau de confiance par les secondes données d'impédance, et l'addition des premières données et des secondes données pour obtenir des données d'impédance fusionnées ;
la détermination, lorsque les données d'impédance fusionnées sont conformes à une distribution normale, d'une moyenne des données d'impédance fusionnées en tant qu'impédance de contact.

**10.** Le procédé selon l'une quelconque des revendications 1 à 9, comprenant en outre :
la vérification du signal EEG après que le signal EEG a été acquis par l'électrode d'acquisition de signal (03), dans lequel ladite vérification du signal EEG comprend :

la détermination (S1110), sur la base d'un signal d'interférence de fréquence d'alimentation qui interfère avec le signal EEG, de premières données d'invalidité pour caractériser la validité du signal EEG ;
la détermination (S1120), sur la base de l'impédance de contact, de secondes données d'invalidité pour caractériser la validité du signal EEG ; et
la réalisation (S1130) d'une fusion de données sur les premières données d'invalidité et les secondes données d'invalidité, pour obtenir des données d'invalidité complètes.

**11.** Le procédé selon la revendication 10, dans lequel ladite détermination (S1110), sur la base du signal d'interférence de fréquence d'alimentation qui interfère avec le signal EEG, des premières données d'invalidité pour caractériser la validité du signal EEG, comprend :

la détermination des premières données d'invalidité, basée sur une intensité de signal du signal d'interférence de fréquence d'alimentation et d'une première relation prédéterminée, la première relation prédéterminée caractérisant une relation entre l'intensité de signal du signal d'interférence de fréquence d'alimentation et les premières données d'invalidité ;
ou dans lequel ladite détermination, sur la base de l'impédance de contact, des secondes données d'invalidité pour caractériser la validité du signal EEG, comprend :
la détermination des secondes données d'invalidité, basée sur l'impédance de contact et d'une seconde relation prédéterminée, la seconde relation prédéterminée caractérisant une relation entre l'impédance de contact et les secondes données d'invalidité.

**12.** Le procédé selon la revendication 11, dans lequel l'intensité de signal du signal d'interférence de fréquence d'alimentation est obtenue par : le filtrage du signal EEG sur la base d'une fréquence prédéterminée pour obtenir un signal EEG filtré, la fréquence prédéterminée comprenant 50 Hz ; l'obtention du signal d'interférence de fréquence d'alimentation sur la base d'une différence entre le signal EEG avant le filtrage et le signal EEG filtré ; et le calcul d'une valeur efficace du signal d'interférence de fréquence d'alimentation en tant qu'intensité de signal du signal d'interférence de fréquence d'alimentation ; et/ou la première relation prédéterminée comprend :

lorsque l'intensité de signal du signal d'interférence de fréquence d'alimentation est inférieure à un premier seuil d'intensité de signal, une tendance selon laquelle les premières données d'invalidité augmentent à mesure que l'intensité de signal du signal d'interférence de fréquence d'alimentation augmente constitue une première tendance ;
lorsque l'intensité de signal du signal d'interférence de fréquence d'alimentation est supérieure ou égale au premier seuil d'intensité de signal et inférieure à un deuxième seuil d'intensité de signal, une tendance selon laquelle les premières données d'invalidité augmentent à mesure que l'intensité de signal du signal d'interférence de fréquence d'alimentation augmente constitue une deuxième tendance ; et
lorsque l'intensité de signal du signal d'interférence de fréquence d'alimentation est supérieure ou égale au deuxième seuil d'intensité de signal et inférieure à un troisième seuil d'intensité de signal, une tendance selon laquelle les premières données d'invalidité augmentent à mesure que l'intensité de signal du signal d'interférence de fréquence d'alimentation augmente constitue une troisième tendance, dans lequel : le premier seuil d'intensité de signal est inférieur au deuxième seuil d'intensité de signal, et le deuxième seuil d'intensité de signal est inférieur au troisième seuil d'intensité de signal ; et la première tendance est inférieure à la deuxième tendance, et la deuxième tendance est inférieure à la troisième tendance ;
ou dans lequel la deuxième relation prédéterminée comprend :

lorsque l'impédance de contact est inférieure à un premier seuil d'impédance, une tendance selon laquelle les secondes données d'invalidité augmentent à mesure que l'impédance de contact augmente constitue une quatrième tendance ;
lorsque l'impédance de contact est supérieure ou égale au premier seuil d'impédance et inférieure à un deuxième seuil d'impédance, une tendance selon laquelle les secondes données d'invalidité augmentent à mesure que l'impédance de contact augmente constitue une cinquième tendance ;
lorsque l'impédance de contact est supérieure ou égale au deuxième seuil d'impédance et inférieure à un troisième seuil d'impédance, une tendance selon laquelle les secondes données d'invalidité augmentent à mesure que l'impédance de contact augmente constitue une sixième tendance, dans lequel : le premier seuil d'impédance est inférieur au deuxième seuil d'impédance, et le deuxième seuil d'impédance est inférieur au troisième seuil d'impédance ; et la quatrième tendance est inférieure à la cinquième tendance, et la cinquième tendance est inférieure à la sixième tendance.

13. Le procédé selon l'une quelconque des revendications 10 à 12, dans lequel ladite réalisation (S1130) de la fusion de données sur les premières données d'invalidité et les secondes données d'invalidité pour obtenir les données d'invalidité complètes, comprend :

la détermination d'un premier poids des premières données d'invalidité ;
la détermination d'un second poids des secondes données d'invalidité ;
l'obtention de premières données en multipliant les premières données d'invalidité par le premier poids, l'obtention de secondes données en multipliant les secondes données d'invalidité par le second poids, et l'addition des premières données et des secondes données pour obtenir les données d'invalidité complètes, dans lequel :

lorsque l'intensité de signal du signal d'interférence de fréquence d'alimentation est supérieure à un seuil prédéterminé, le premier poids est supérieur au second poids ;
lorsque l'intensité de signal du signal d'interférence de fréquence d'alimentation est inférieure ou égale au seuil prédéterminé, le premier poids est inférieur ou égal au second poids ; et
la somme du premier poids et du second poids est égale à 1.

14. Un dispositif électronique (1400) pour commander une électrode d'acquisition de signal (03), dans lequel l'électrode d'acquisition de signal (03) est connectée de manière mobile à un composant portable (01) d'un appareil d'acquisition de signal d'électroencéphalogramme (EEG), le composant portable (01) étant adapté pour être porté sur la tête d'un

**EP 4 578 386 B1**

utilisateur, et l'appareil d'acquisition de signal EEG comprenant en outre un composant de poussée (02), et dans lequel le dispositif électronique (1400) comprend :

> une mémoire (1403) ;
> un processeur (1401) ; et
> un programme informatique stocké dans la mémoire (1403) et exécutable par le processeur (1401), dans lequel le processeur (1401), lorsqu'il exécute le programme informatique, met en œuvre le procédé selon l'une quelconque des revendications 1 à 13.

EP 4 578 386 B1

FIG. 1

FIG. 2

| Controller 05 | Pushing component 02 |
|---|---|
| Impedance detection component 06 | Signal acquisition electrode 03 |

FIG. 3

Obtain a contact impedance between the signal acquisition electrode and the head of the user — S401

Detect whether the contact impedance is greater than or equal to a first impedance threshold — S402 → End

Control the pushing component to push the signal acquisition electrode to move towards the head of the user — S403

FIG. 4

FIG. 5

FIG. 6

FIG. 7

FIG. 8

Weight

First weight

Second
weight

K

Signal intensity of power frequency
interference signal

FIG. 9

1000

S1010

Apply, by using a first alternating-current constant current source, an electrical
signal to the signal acquisition electrode, and detect first impedance data between
the signal acquisition electrode and the head

S1020

Apply, by using a second alternating-current constant current source, an electrical
signal to the signal acquisition electrode, and detect second impedance data between
the signal acquisition electrode and the head

S1030

Determine, based on a data distribution feature of the first impedance data and a
data distribution feature of the second impedance data, a first confidence level of
the first impedance data and a second confidence level of the second impedance
data

S1040

Perform data fusion on the first impedance data and the second impedance data
based on the first confidence level and the second confidence level, to obtain the
contact impedance

FIG. 10

1100

S1110

Determine, based on a power frequency interference signal that interferes with the EEG signal, first invalidity data for characterizing validity of the EEG signal

S1120

Determine, based on the contact impedance, second invalidity data for characterizing validity of the EEG signal

S1130

Perform data fusion on the first invalidity data and the second invalidity data, to obtain comprehensive invalidity data

FIG. 11

1210

First detection module

1220

Second detection module

1230

First determination module

1240

First fusion module

1200

FIG. 12

FIG. 13

FIG. 14

**EP 4 578 386 B1**

**REFERENCES CITED IN THE DESCRIPTION**

**Patent documents cited in the description**

- CN 202311814246
- CN 202311810635
- CN 202311808091
- WO 2013038285 A1
- US 2019000338 A1
- US 2011015503 A1
- CN 112315483 A